# EUROPÄISCHE PATENTSCHRIFT

(11) **EP 3 752 107 B1**
(45) Veröffentlichungstag und Bekanntmachung des Hinweises auf die Patenterteilung: **25.09.2024**
(21) Anmeldenummer: 19705492.7
(22) Anmeldetag: 14.02.2019
(51) Int. Cl.: A61F 5/01

(54) **TRAGBARE SITZHALTUNGSHILFEVORRICHTUNG**
WEARABLE SITTING-POSTURE AID
DISPOSITIF PORTATIF D'AIDE À LA POSITION ASSISE

(30) Priorität: 14.02.2018 DE 102018103302
(43) Veröffentlichungstag der Anmeldung: 23.12.2020
(73) Patentinhaber: noonee AG, 8008 Zürich (CH)
(72) Erfinder: VAFI, Daniel, 8006 Zürich (CH)
(74) Vertreter: Daub, Thomas
(86) Internationale Anmeldenummer: PCT/EP2019/053621
(87) Internationale Veröffentlichungsnummer: WO 2019/158623

(56) Entgegenhaltungen:
- EP-A1- 3 158 894
- CN-A- 106 377 394
- US-A- 4 138 156
- US-A- 4 641 882

## Beschreibung

### Stand der Technik

Die Erfindung betrifft eine tragbare Sitzhaltungshilfevorrichtung gemäß dem Oberbegriff des Patentanspruchs 1, eine Fußeinheit gemäß dem Oberbegriff des Patentanspruchs 11 sowie ein Verfahren zum Betrieb der tragbaren Sitzhaltungshilfevorrichtung gemäß dem Oberbegriff des Patentanspruchs 12.

Es ist bereits eine tragbare Sitzhaltungshilfevorrichtung vorgeschlagen worden.

EP3 158 894 wird als nächstliegenden Stand der Technik angesehen und offenbart eine Steuereinheit für eine tragbare Vorrichtung zur Unterstützung der Sitzhaltung. Die Erfindung betrifft eine Steuereinheit für eine tragbare Sitzhaltungshilfevorrichtung mit mindestens einem Betriebsprogramm.

Die Aufgabe der Erfindung besteht insbesondere darin, eine gattungsgemäße Vorrichtung und/oder ein Verfahren zum Betrieb der Vorrichtung mit verbesserten Eigenschaften hinsichtlich eines Komforts, einer Ergonomie und/oder einer Physiologie bereitzustellen.

Die Aufgabe wird insbesondere erfindungsgemäß durch die Merkmale des Patentanspruchs 1, des Patentanspruchs 11 und/oder des Patentanspruchs 12 gelöst, während vorteilhafte Ausgestaltungen und Weiterbildungen der Erfindung den Unteransprüchen entnommen werden können.

### Vorteile der Erfindung

Die Erfindung geht aus von einer tragbaren Sitzhaltungshilfevorrichtung mit zumindest einer Beineinheit, welche zumindest eine Beinlängsachse, insbesondere eine obere und/oder eine untere Beinlängsachse, definiert, und mit zumindest einer Fußeinheit, welche zur Anbindung eines Schuhs und/oder eines Fußes einer Person vorgesehen ist, wobei die Fußeinheit zumindest einen Schuhadapter und zumindest eine Schuhbindung umfasst, welche zumindest quer zu der Beinlängsachse, insbesondere der oberen und/oder der unteren Beinlängsachse, miteinander koppelbar sind.

Es wird vorgeschlagen, dass die die tragbare Sitzhaltungshilfevorrichtung eine Schnellkopplung umfasst, welche dazu vorgesehen ist, den Schuhadapter und die Schuhbindung miteinander werkzeuglos zu koppeln, wobei die Schnellkopplung zumindest ein Rastelement umfasst, welches zumindest teilweise zur Ausbildung einer Kopplung des Schuhadapters mit der Schuhbindung vorgesehen ist. Hierdurch kann ein Komfort, insbesondere ein Tragekomfort, und/oder eine Ergonomie verbessert werden. Insbesondere kann der Tragekomfort erhöht werden, indem mittels der Fußeinheit eine schnelle Kopplung und/oder Entkopplung der Füße eines Benutzers, und zwar insbesondere sowohl in einer Sitzhaltung als auch in einer Stehhaltung als auch beim Gehen, vereinfacht wird. Insbesondere kann der Tragekomfort in einer Sitzhaltung und/oder Stehhaltung verbessert werden, da die Füße eines Benutzers in individuellen Positionen, also insbesondere in der Sitzhaltung, in der Stehhaltung und/oder beim Gehen, gekoppelt und/oder entkoppelt werden können. Ferner kann vorzugsweise der Tragekomfort beim Gehen verbessert werden, da es eine schnelle Entkopplung bzw. Einkopplung bei einem Wechsel zwischen Gehen und Sitzhaltung erzielt werden kann. Zudem kann eine Sicherheit, insbesondere eine Betriebs und/oder Nutzungssicherheit, verbessert werden. Insbesondere kann vermieden werden, dass Gelenke und/oder Körperteile ungewollt belastet und/oder durch die tragbare Sitzhaltungshilfevorrichtung blockiert werden, indem bei einer Überlast auf Körperteile diese von der tragbaren Sitzhaltungshilfevorrichtung schnell entkoppelt werden können. Durch die Schnellkopplung kann vorteilhaft ein Komfort, insbesondere ein Benutzerkomfort, weiter verbessert werden, da auf weitere Werkzeuge zur Kopplung verzichtet werden kann. Insbesondere kann eine Sicherheit verbessert werden, da auch in Notsituationen eine Kopplung und/oder Entkopplung werkzeuglos erfolgen kann.

Unter einer "Schnellkopplung" soll insbesondere eine vorzugsweise mechanische und/oder magnetische Einheit verstanden werden, welche dazu vorgesehen ist, zumindest zwei Bauteile werkzeuglos, zerstörungsfrei und/oder wiederholbar miteinander vorzugsweise einhändig, einbeinig und/oder einfüßig zu koppeln, und zwar ganz besonders vorteilhaft mit einer einzigen Handbewegung, Fußbewegung und/oder Beinbewegung einer Person. Beispielsweise kann eine Schnellkopplung einen Klettverschluss, einen Magnetverschluss, einen Klipp- und/oder Rastverschluss, einen Drehverschluss und/oder einen Bajonettverschluss oder dergleichen ausbilden. Vorzugsweise weist die Schnellkopplung eine Kombination von Magnetverschluss und Rastverschluss auf. Insbesondere zu einer Kopplung weist die Schnellkopplung zumindest ein Schnellkoppelelement und zumindest ein weiteres Schnellkoppelelement, welches zu dem Schnellkoppelelement korrespondierend ausgebildet ist, auf. Das Schnellkoppelelement ist insbesondere an dem Schuhadapter angeordnet. Das weitere Schnellkoppelelement ist insbesondere an der Schuhbindung angeordnet. Insbesondere kann die Schnellkopplung zumindest teilweise einstückig mit der Fußeinheit, insbesondere mit dem Schuhadapter und/oder der Schuhbindung, ausgebildet sein. Vorzugsweise könnte das Schnellkoppelelement zumindest teilweise einstückig mit dem Schuhadapter ausgebildet sein. Insbesondere könnte das weitere Schnellkoppelelement zumindest teilweise einstückig mit der Schuhbindung ausgebildet sein.

Unter einem "Rastelement" soll insbesondere ein vorzugsweise elastisches Element verstanden werden, welches dazu vorgesehen ist, zumindest eine Rastverbindung herzustellen, wie beispielsweise mittels eines Kraft- und/oder Formschlusses. Insbesondere weist das weitere Schnellkoppelelement das Rastelement auf. Alternativ könnte das Schnellkoppelelement einstückig mit dem Rastelement ausgebildet sein. Das Rastelement weist vorzugsweise einen beispielsweise konusförmigen Vorsprung auf. Ferner weist die Schnellkopplung insbesondere zumindest ein weiteres Rastelement auf, welches vorzugsweise zu dem Rastelement korrespondierend ausgebildet ist. Das weitere Schnellkoppelelement weist insbesondere das weitere Rastelement auf. Alternativ könnte das weitere Schnellkoppelelement einstückig mit dem weiteren Rastelement ausgebildet sein. Insbesondere zur Kopplung ist das weitere Rastelement dazu vorgesehen, das Rastelement, insbesondere den Vorsprung, zumindest teilweise zu hintergreifen.

In einem weiteren Aspekt der Erfindung wird vorgeschlagen, dass die tragbare Sitzhaltungshilfevorrichtung zumindest eine Lagereinheit umfasst, welche zumindest einen Teil der Fußeinheit zumindest quer zu der zumindest einen Beinlängsachse, insbesondere der oberen und/oder der unteren Beinlängsachse, translatorisch bewegbar lagert. Hierdurch kann ein Komfort, insbesondere ein Tragekomfort, eine Ergonomie und/oder eine Physiologie verbessert werden. Insbesondere kann der Tragekomfort erhöht werden, indem mittels der Lagereinheit eine Bewegungsfreiheit eines Benutzers, und zwar insbesondere sowohl in einer Sitzhaltung als auch in einer Stehhaltung als auch beim Gehen, erweitert wird. Insbesondere kann der Tragekomfort in einer Sitzhaltung und/oder Stehhaltung verbessert werden, da die Lagereinheit eine individuelle Positionierung der Fußeinheit und damit der Füße eines Benutzers ermöglicht. Ferner kann vorzugsweise der Tragekomfort beim Gehen verbessert werden, da die Lagereinheit ein für ein Gehen notwendiges Strecken und Zusammenziehen der Füße relativ zum Bein ermöglicht. Somit kann insbesondere beim Gehen eine Physiologie der tragbaren Sitzhaltungshilfevorrichtung verbessert werden. Zudem kann insbesondere eine Sicherheit, insbesondere eine Betriebs- und/oder Nutzungssicherheit, verbessert werden. Insbesondere mittels der Lagereinheit kann vermieden werden, dass Bewegungsabläufe, Gelenke und/oder Körperteile in ungewollter Weise blockiert und/oder überlastet werden.

Unter einer "tragbaren Sitzhaltungshilfevorrichtung" soll hier insbesondere eine Vorrichtung verstanden werden, welche dazu vorgesehen ist, zumindest teilweise, vorzugsweise zumindest zu einem Großteil und besonders bevorzugt vollständig eine Gewichtskraft einer in einer Sitzhaltung befindlichen oder in einer teilweisen Sitzhaltung befindlichen Person aufzunehmen und die Gewichtskraft zumindest teilweise, vorzugsweise zumindest zu einem Großteil und besonders bevorzugt vollständig auf einen Untergrund zu übertragen. Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs-und/oder Betriebszustand erfüllt und/oder ausführt. Unter dem Ausdruck "zumindest zu einem Großteil" soll dabei insbesondere zumindest zu 55 %, vorzugsweise zumindest zu 65 %, bevorzugt zumindest zu 75 %, besonders bevorzugt zumindest zu 85 % und ganz besonders bevorzugt zumindest zu 95 % verstanden werden. Die tragbare Sitzhaltungshilfevorrichtung ermöglicht insbesondere der Person, von welcher sie getragen wird, beliebig nach Bedarf umherzugehen, zu stehen und/oder sich auf die tragbare Sitzhaltungshilfevorrichtung zu setzen sowie aufzustehen, nachdem sie auf der tragbaren Sitzhaltungshilfevorrichtung gesessen oder teilweise gesessen ist. Insbesondere beträgt ein Winkel zwischen einem Oberschenkel und einem Unterschenkel der Person in der Sitzhaltung höchstens 130°, vorzugsweise höchstens 120° und besonders bevorzugt höchstens 110° und/oder insbesondere mindestens 60°, bevorzugt mindestens 70° und besonders bevorzugt mindestens 80°. Vorzugsweise beträgt der Winkel zwischen dem Oberschenkel und dem Unterschenkel der Person in der Sitzhaltung zumindest im Wesentlichen 90°. Unter "zumindest im Wesentlichen" soll insbesondere eine Berücksichtigung einer Abweichung von einem angegebenen Wert um höchstens 15 %, vorzugsweise höchstens 10 %, bevorzugt höchstens 5 % und ganz besonders bevorzugt um höchstens 1 % verstanden werden. Insbesondere beträgt ein Winkel zwischen einem Oberschenkel und einem Unterschenkel der Person in der teilweisen Sitzhaltung höchstens 170°, vorzugsweise höchstens 160° und besonders bevorzugt höchstens 150° und/oder insbesondere mindestens 100°, bevorzugt mindestens 110° und besonders bevorzugt mindestens 120°. Vorzugsweise beträgt der Winkel zwischen dem Oberschenkel und dem Unterschenkel der Person in der teilweisen Sitzhaltung zumindest im Wesentlichen 130°. Unter einer "teilweisen Sitzhaltung" soll insbesondere eine Haltung verstanden werden, in welcher sich eine Person nach vorne lehnt und gleichzeitig teilweise ihre Knie beugt. Insbesondere kann eine die tragbare Sitzhaltungshilfevorrichtung tragende Person sitzen und/oder teilweise sitzen und/oder sich auf die tragbare Sitzhaltungshilfevorrichtung setzen, wobei die tragbare Sitzhaltungshilfevorrichtung zumindest teilweise, vorzugsweise zumindest einem Großteil und besonders bevorzugt vollständig der Gewichtskraft entgegenwirkt und/oder wobei die Person nur einem Bruchteil ihrer Gewichtskraft mit Muskelkraft entgegenwirkt. Unter einem "Bruchteil" soll insbesondere ein Teil einer Gesamtheit verstanden werden, welcher höchstens 15 %, vorzugsweise höchstens 10 % und besonders bevorzugt höchstens 5 % der Gesamtheit beträgt. Insbesondere ist die tragbare Sitzhaltungshilfevorrichtung dazu vorgesehen, von der Person getragen zu werden, während die Person steht und/oder während die Person geht. Darunter, dass "ein Objekt von einer Person getragen wird", soll insbesondere verstanden werden, dass das Objekt von einer Person am Körper getragen wird und/oder am Körper angelegt ist, wie dies beispielsweise bei einem Kleidungsstück und/oder einer Arbeitsausrüstung der Fall ist. Vorteilhafterweise ist die tragbare Sitzhaltungshilfevorrichtung dazu vorgesehen, verschiedene Sitzhaltungen und/oder teilweise Sitzhaltungen, welche insbesondere durch verschiedene Sitzwinkel definiert sind, zu unterstützen.

Die tragbare Sitzhaltungshilfevorrichtung definiert insbesondere eine Sitzrichtung. Vorzugsweise schaut die Person in die Sitzrichtung und/oder ist der Sitzrichtung zugewandt, wenn sie auf der tragbaren Sitzhaltungshilfevorrichtung sitzt oder teilweise sitzt und nach vorne gewandt ist. Insbesondere ist die Sitzrichtung parallel zu einem Boden ausgerichtet, oberhalb dessen die Person sitzt und/oder auf dem die Person steht und/oder geht, während sie die tragbare Sitzhaltungshilfevorrichtung trägt. Insbesondere definiert die tragbare Sitzhaltungshilfevorrichtung eine Gehrichtung. Vorzugsweise ist die Person in die Gehrichtung gewandt, wenn sie mit der tragbaren Sitzhaltungshilfevorrichtung geht und/oder steht und nach vorne gewandt ist. Insbesondere ist die Gehrichtung parallel zu einem Boden ausgerichtet, oberhalb dessen die Person sitzt und/oder auf dem die Person steht und/oder geht, während sie die tragbare Sitzhaltungshilfevorrichtung trägt. Insbesondere ist die tragbare Sitzhaltungshilfevorrichtung lediglich dazu ausgerichtet, die Gewichtskraft aufzunehmen und zu übertragen. Vorzugsweise ist die tragbare Sitzhaltungshilfevorrichtung nicht dazu ausgelegt, eine steuerbare Kraft zu erzeugen, welche dazu vorgesehen ist, eine Person zu unterstützen, wenn sie geht, steht und/oder Lasten hebt.

Die Beineinheit weist insbesondere zumindest ein, vorzugsweise ein oberes Beinteil und/oder zumindest ein, vorzugsweise ein unteres Beinteil, auf. Das obere Beinteil ist insbesondere einem Oberschenkel einer Person, welche die Sitzhaltungshilfevorrichtung trägt, zugeordnet und/oder an diesem befestigt. Eine Haupterstreckung des unteren Beinteils definiert eine untere Beinlängsachse. Das untere Beinteil ist insbesondere einem Unterschenkel einer Person, welche die Sitzhaltungshilfevorrichtung trägt, zugeordnet und/oder an diesem befestigt. Insbesondere sind das obere Beinteil und das untere Beinteil relativ zueinander beweglich miteinander verbunden. Eine Haupterstreckung des oberen Beinteils, definiert eine obere Beinlängsachse. Vorteilhaft weist die Beineinheit zumindest ein, vorzugsweise ein einziges Kniegelenk auf, welches das obere Beinteil und das untere Beinteil miteinander verbindet.

Die Fußeinheit ist insbesondere mit der Beineinheit, insbesondere mit dem unteren Beinteil verbunden. Die Schuhbindung der Fußeinheit ist insbesondere dazu vorgesehen, einen Schuh und/oder einen Fuß mit der Beineinheit zu koppeln und/oder zu verbinden. Darunter, dass "zwei Bauteile koppelbar" sind, soll insbesondere verstanden werden, dass diese miteinander verbindbar und insbesondere wiederholbar, vorzugsweise zerstörungsfrei wieder voneinander trennbar sind. Eine Kopplung zweier Bauteile kann etwa durch einen Kraft- und/oder Formschluss hervorgerufen werden. Unter "kraft-und/oder formschlüssig verbunden" soll dabei insbesondere lösbar verbunden verstanden werden, wobei eine Haltekraft zwischen den zwei Bauteilen vorzugsweise durch einen geometrischen Eingriff der Bauteile ineinander und/oder durch eine Reibkraft zwischen den Bauteilen übertragen wird. Eine Kopplung zweier Bauteile schließt insbesondere eine stoffschlüssige Verbindung der Bauteile miteinander aus. Unter "stoffschlüssig verbunden" soll insbesondere verstanden werden, dass die Masseteile durch atomare oder molekulare Kräfte zusammengehalten werden, wie beispielsweise beim Löten, Schweißen, Kleben und/oder Vulkanisieren. Insbesondere sind der Schuhadapter und die Schuhbindung entlang einer Koppelrichtung, welche quer zur Beinlängsachse, insbesondere der obere und/oder der untere Beinlängsachse, verläuft, miteinander koppelbar, indem diese aufeinander und/oder ineinander entlang der Koppelrichtung Richtung geführt werden. Insbesondere ist die Koppelrichtung zumindest im Wesentlichen senkrecht zu einer Expansionsrichtung und/oder Kontraktionsrichtung der Lagereinheit.

Die Schuhbindung ist insbesondere dazu vorgesehen, an einem Schuh und/oder einem Fuß der Person getragen zu werden. Die Schuhbindung weist vorteilhaft zumindest ein Schuhband auf. Insbesondere weist die Schuhbindung zumindest ein oberes Band auf, welches vorteilhaft quer über einen Spann des Fußes oder Schuhs, an welchen die Fußeinheit angebunden ist, verläuft. Vorzugsweise weist die Schuhbindung zumindest ein unteres Band auf, welches vorteilhaft quer über eine Sohle des Fußes oder Schuhs verläuft, welcher mittels der Fußeinheit angebunden ist. Unter "quer" soll insbesondere eine Anordnung verstanden werden, welche von einer zumindest im Wesentlichen parallelen Anordnung verschieden ist. Vorzugsweise soll unter einer queren Anordnung eine zumindest im Wesentlichen senkrechte Anordnung verstanden werden. Insbesondere trägt die Person in einem Schuhbindungstragezustand die Schuhbindung an dem Schuh und/oder an dem Fuß. Die Schuhbindung ist in dem Schuhbindungstragezustand vorzugsweise an dem Schuh und/oder an dem Fuß der Person angebracht. Insbesondere befindet sich die Schuhbindung in dem normalen Tragezustand in einem Schuhbindungstragezustand. Die Fußeinheit weist vorteilhaft zumindest einen Schuhadapter auf. Der Schuhadapter ist vorzugsweise mit der Schuhbindung koppelbar ausgebildet. Mittels des Schuhadapters ist die Schuhbindung somit mit der Beineinheit verbindbar. Der Teil der Fußeinheit, welcher translatorisch bewegbar gelagert ist, ist insbesondere zumindest der Schuhadapter und/oder die Schuhbindung der Fußeinheit.

Vorteilhaft weist die Fußeinheit zumindest eine Fußeinheitsstütze auf. Die Fußeinheitsstütze ist vorzugsweise als ein Bügel ausgebildet. Es ist denkbar, dass die Fußeinheitsstütze zumindest teilweise einstückig oder einstückig mit dem unteren Beinteil, insbesondere mit der unteren Beinstütze, ausgebildet ist. Insbesondere ist die Lagereinheit an der Fußeinheitsstütze angeordnet oder zumindest teilweise einstückig mit dieser ausgebildet. Die Lagereinheit der Fußeinheit ist eine Einheit, welche dazu vorgesehen ist, ein Lager auszubilden. Insbesondere weist die Lagereinheit zumindest ein Lagerelement und zumindest ein weiteres Lagerelement auf, welche zumindest zu einer Ausbildung des Lagers vorgesehen sind und einander vorzugsweise gegenseitig führen. Denkbar ist, dass die Lagereinheit als ein Gleitlager ausgebildet ist, wobei insbesondere das Lagerelement und das weitere Lagerelement zueinander korrespondierend ausgebildet und sich gegenseitig kontaktierend zu einer Gleitlagerung ausgebildet sind. Insbesondere weist das Lagerelement zumindest ein Führungselement auf und das weitere Lagerelement weist ein korrespondierendes Führungselement auf, welche einander vorzugsweise zumindest teilweise umgreifen. Die Führungselemente sind insbesondere als korrespondierende Führungsschienen ausgebildet. Alternativ könnte die Lagereinheit als ein Wälzlager ausgebildet sein, wobei die Lagereinheit vorzugsweise zusätzlich zur Ausbildung des Lagers zumindest ein, vorzugsweise mehrere Wälzelemente umfasst, welche zwischen dem Lagerelement und dem weiteren Lagerelement und insbesondere deren jeweiligen Führungselementen angeordnet sind, um diese aufeinander abwälzend zu lagern. Beispielsweise könnte ein solches Wälzelement als eine Kugel, eine Rolle oder dergleichen ausgebildet sein. Insbesondere ist das Lagerelement relativ zu dem weiteren Lagerelement zumindest verschiebbar. Das Lagerelement und das weitere Lagerelement sind translatorisch zueinander bewegbar. Unter "translatorisch" soll insbesondere eine Art einer Bewegung eines Objekts verstanden werden, bei welcher alle Punkte des Objekts dieselbe Verschiebung erfahren, wobei vorzugsweise Geschwindigkeiten und Beschleunigungen aller Punkte identisch sind. Insbesondere ist eine translatorische Bewegung verschieden von einer rotatorischen Bewegung. Insbesondere ist zumindest der Schuhadapter und/oder die Schuhbindung an der Lagereinheit angeordnet und/oder mit dieser verbunden. Alternativ oder zusätzlich ist denkbar, dass der Schuhadapter und/oder die Schuhbindung vorzugsweise jeweils zumindest teilweise einstückig mit der Lagereinheit ausgebildet sind. Beispielsweise könnte das Lagerelement zumindest teilweise einstückig mit dem Schuhadapter ausgebildet sein. Ferner könnte beispielsweise das weitere Lagerelement zumindest teilweise einstückig mit der Schuhbindung ausgebildet sein.

Ferner weist die Beineinheit insbesondere zumindest eine Bodenkontakteinheit auf. Insbesondere umfasst die Bodenkontakteinheit zumindest ein Bodenkontaktelement. Das Bodenkontaktelement weist vorzugsweise zumindest eine Bodenkontaktfläche auf, welche vorteilhaft dazu vorgesehen ist, einen Boden zu kontaktieren, wenn die Person auf der tragbaren Sitzhaltungshilfevorrichtung sitzt oder teilweise sitzt. Die Bodenkontaktfläche ist vorteilhaft gebogen und/oder gekrümmt, insbesondere konvex gebogen und/oder konvex gekrümmt. Bevorzugt ist zumindest ein Teil des Bodenkontaktelements oder das ganze Bodenkontaktelement ellipsoidförmig und/oder rotationsellipsoidförmig und/oder kugelförmig. Das Bodenkontaktelement besteht insbesondere zumindest teilweise, vorzugsweise zumindest großteils, vorteilhaft vollständig aus Gummi. Vorzugsweise wird eine Gewichtskraft der Person von der Sitzeinheit zu der oberen Beinstütze und/oder von der oberen Beinstütze zum Kniegelenk und/oder vom Kniegelenk zu der unteren Beinstütze und/oder von der unteren Beinstütze zu dem Bodenkontaktelement und/oder von dem Bodenkontaktelement zum Boden übertragen. Insbesondere wird die Gewichtskraft der Person zusätzlich über den Fuß oder den Schuh der Person zum Boden übertragen. Wenn die Person auf der tragbaren Sitzhaltungshilfevorrichtung sitzt oder teilweise sitzt, steht, zusätzlich zum Bodenkontaktelement, der Fuß und/oder der Schuh der Person in Kontakt mit dem Boden. Bevorzugt ist das Bodenkontaktelement relativ zum Boden kontaktfrei angeordnet, wenn die Person geht oder steht, während sie die tragbare Sitzhaltungshilfevorrichtung trägt. Die Bodenkontakteinheit ist insbesondere mit dem unteren Beinteil und/oder der Fußeinheit verbunden. Es ist denkbar, dass die Bodenkontakteinheit zumindest teilweise einstückig mit dem unteren Beinteil und/oder zumindest teilweise einstückig mit der Fußeinheit ausgebildet ist. Es ist ebenfalls denkbar, dass die Fußeinheit zumindest teilweise einstückig mit dem unteren Beinteil ausgebildet ist. Unter "zumindest teilweise einstückig verbunden und/oder ausgebildet" soll in diesem Zusammenhang insbesondere verstanden werden, dass ein Objekt zumindest ein Bauteil aufweist, welches einstückig mit zumindest einem weiteren Bauteil des Objekts verbunden ist. Unter "einstückig" soll insbesondere zumindest stoffschlüssig verbunden verstanden werden, beispielsweise durch einen Schweißprozess, einen Klebeprozess, einen Anspritzprozess und/oder einen anderen, dem Fachmann als sinnvoll erscheinenden Prozess. Vorteilhaft soll unter einstückig auch einteilig verstanden werden. Unter "einteilig" soll insbesondere in einem Stück geformt verstanden werden, wie beispielsweise durch eine Herstellung aus einem Guss und/oder durch eine Herstellung in einem Ein- oder Mehrkomponentenspritzverfahren und vorteilhaft aus einem einzelnen Rohling.

Die tragbare Sitzhaltungshilfevorrichtung umfasst insbesondere zumindest eine Oberkörpertrageeinheit. Insbesondere ist die Beineinheit mit der Oberkörpertrageeinheit verbunden, und zwar vorzugsweise mittels zumindest eines Verbindungsbands. Vorzugsweise trägt die die tragbare Sitzhaltungshilfevorrichtung tragende Person die Oberkörpertrageeinheit an ihrem Oberkörper. Vorteilhaft ist die Oberkörpertrageeinheit als Gürtel und/oder als Hosenträger und/oder als Anschnaller ausgebildet.

Die tragbare Sitzhaltungshilfevorrichtung umfasst insbesondere zumindest eine zusätzliche Beineinheit. Die tragbare Sitzhaltungshilfevorrichtung weist vorzugsweise je Bein einer Person zumindest eine, vorzugsweise genau eine, Beineinheit auf. Insbesondere umfasst die tragbare Sitzhaltungshilfevorrichtung zwei, vorteilhaft genau zwei, Beineinheiten. Vorteilhafterweise sind die Beineinheit und die zusätzliche Beineinheit identisch ausgebildet. Es ist ebenfalls denkbar, dass die Beineinheit und die zusätzliche Beineinheit spiegelsymmetrisch zueinander ausgebildet sind. Es ist denkbar, dass die Beineinheit dazu vorgesehen ist, an einem linken Bein getragen zu werden und die zusätzliche Beineinheit dazu vorgesehen ist, an einem rechten Bein getragen zu werden, oder umgekehrt. Vorteilhaft ist die Beineinheit dazu vorgesehen, entweder an einem linken Bein oder an einem rechten Bein getragen zu werden. Weiter vorteilhaft ist die zusätzliche Beineinheit dazu vorgesehen, an einem rechten Bein getragen zu werden. Vorzugsweise ist die zusätzliche Beineinheit mit der Oberkörpertrageeinheit verbunden, bevorzugt mittels zumindest eines Verbindungsbands. Insbesondere trägt die die tragbare Sitzhaltungshilfevorrichtung tragende Person die Beineinheit, insbesondere nur die Beineinheit, an einem ersten Bein, beispielsweise einem linken Bein oder einem rechten Bein. Insbesondere trägt die die tragbare Sitzhaltungshilfevorrichtung tragende Person die zusätzliche Beineinheit, insbesondere nur die zusätzliche Beineinheit, an einem zweiten Bein, beispielsweise einem rechten Bein oder einem linken Bein. Vorteilhaft ist die Beineinheit auf einer Rückseite des Beins angeordnet, an dem die Beineinheit getragen wird. Weiter vorteilhaft ist die zusätzliche Beineinheit an einer Rückseite des Beins angeordnet, an dem die zusätzliche Beineinheit getragen wird. Insbesondere sind die Beineinheiten der tragbaren Sitzhaltungshilfevorrichtung auf einer Rückseite der Beine der Person angeordnet, während die Person auf der tragbaren Sitzhaltungshilfevorrichtung sitzt und/oder teilweise sitzt und/oder mit der tragbaren Sitzhaltungshilfevorrichtung steht und/oder geht.

Für eine Verbindung mit einem Oberschenkel der Person umfasst das obere Beinteil bevorzugt zumindest eine Oberschenkelverbindungseinheit. Vorzugsweise weist die Oberschenkelverbindungseinheit zumindest ein Oberschenkelband auf. Insbesondere umfasst das obere Beinteil eine Sitzeinheit, welche dazu vorgesehen ist, insbesondere falls die Person auf der tragbaren Sitzhaltungshilfevorrichtung sitzt oder teilweise sitzt, eine Sitzfläche für die Person, vorzugsweise für den Oberschenkel und/oder zumindest einen unteren Teil einer Gesäßbacke der Person, bereitzustellen. Vorzugsweise umfasst die Sitzeinheit zumindest ein Sitzelement, welches die Sitzfläche aufweist. Vorteilhaft steht die Sitzeinheit in Kontakt mit dem Oberschenkel der Person, wenn die Person auf der tragbaren Sitzhaltungshilfevorrichtung sitzt oder teilweise sitzt. Bevorzugt ist die Sitzeinheit auf einer Rückseite des Oberschenkels der Person angeordnet, wenn die Person mit der tragbaren Sitzhaltungshilfevorrichtung steht oder geht.

Das obere Beinteil weist vorteilhaft zumindest eine obere Beinstütze auf. Vorzugsweise ist die Sitzeinheit mit der oberen Beinstütze verbunden. Vorteilhaft ist die Oberschenkelverbindungseinheit und/oder das Oberschenkelband mit der oberen Beinstütze verbunden. Insbesondere ist die obere Beinstütze als Rahmenelement ausgebildet. Vorzugsweise ist die obere Beinstütze als längliches Element ausgebildet. Vorteilhaft weist das obere Beinteil zumindest eine obere Beinlängsachse auf, welche zumindest im Wesentlichen parallel zu einer Längsachse des Oberschenkels der Person ausgerichtet ist. Vorzugsweise ist eine Haupterstreckungsrichtung der oberen Beinstütze zumindest im Wesentlichen parallel zu der oberen Beinlängsachse ausgerichtet. Unter "zumindest im Wesentlichen parallel" soll insbesondere eine Ausrichtung einer Richtung relativ zu einer Bezugsrichtung verstanden werden, wobei die Richtung und die Bezugsrichtung, insbesondere in einer Ebene betrachtet, einen Winkel von 0° einschließen, wobei der Winkel insbesondere eine maximale Abweichung von insbesondere kleiner als 8°, vorteilhaft kleiner als 5° und besonders vorteilhaft kleiner als 2°, aufweist. Insbesondere besteht die obere Beinstütze zumindest teilweise, bevorzugt zumindest zu einem Großteil, vorteilhaft vollständig aus Plastik. Es ist auch denkbar, dass die obere Beinstütze zumindest teilweise, bevorzugt zumindest zu einem Großteil, vorteilhaft vollständig aus einem Leichtmetall oder einer Leichtmetalllegierung, beispielsweise Aluminium und/oder Titan und/oder Beryll und/oder Scandium oder anderen geeigneten Metallen besteht. Des Weiteren ist es denkbar, dass die obere Beinstütze zumindest teilweise, bevorzugt zumindest zu einem Großteil, vorteilhaft vollständig aus einem Verbundwerkstoff besteht, insbesondere einem Faserverbundwerkstoff und/oder einem faserverstärkten Kunststoff und/oder einem Karbonfaserverbundwerkstoff und/oder einem karbonfaserverstärkten Polymer und/oder einem faserverstärkten Thermoplast. Vorzugsweise ist das untere Beinteil auf einer Rückseite des Unterschenkels der Person angeordnet. Insbesondere umfasst das untere Beinteil zumindest eine untere Beinstütze. Vorteilhafterweise ist die untere Beinstütze als Rahmenelement ausgebildet. Bevorzugt ist die untere Beinstütze als längliches Element ausgebildet.

Vorzugsweise definiert eine Ausrichtung des oberen Beinteils und des unteren Beinteils zueinander zusammen einen Sitzwinkel. Der Sitzwinkel ist vorteilhaft ein Winkel, welcher zwischen der oberen Beinlängsachse und der unteren Beinlängsachse, insbesondere auf einer jeweiligen Rückseite des oberen Beinteils und des unteren Beinteils, aufgespannt ist. Insbesondere beträgt der Sitzwinkel in der Sitzhaltung höchstens 130°, vorzugsweise höchstens 120° und besonders bevorzugt höchstens 110°, und/oder insbesondere mindestens 60°, vorzugsweise mindestens 70° und besonders bevorzugt mindestens 80°. Insbesondere beträgt der Sitzwinkel in der Sitzhaltung zumindest im Wesentlichen 90°. Insbesondere beträgt der Sitzwinkel in der teilweisen Sitzhaltung nicht mehr als 170°, vorzugsweise höchstens 160° und vorteilhaft höchstens 150°, und/oder insbesondere wenigstens 100°, vorzugsweise wenigstens 110° und besonders bevorzugt wenigstens 120°. Insbesondere beträgt der Sitzwinkel in der teilweisen Sitzhaltung zumindest im Wesentlichen 130°. Bevorzugt korrespondiert der Sitzwinkel zu dem Winkel zwischen dem Oberschenkel und dem Unterschenkel der Person. Insbesondere beträgt der Sitzwinkel in einer Stehhaltung zumindest im Wesentlichen 180°.

Insbesondere verbindet das Kniegelenk das untere Beinteil schwenkbar, vorzugsweise um eine Kniegelenkachse schwenkbar, mit dem oberen Beinteil. Vorteilhaft ist die Kniegelenkachse zumindest im Wesentlichen senkrecht zu der oberen Beinlängsachse und/oder zu der unteren Beinlängsachse ausgerichtet. Bevorzugt ist die Kniegelenkachse zumindest im Wesentlichen senkrecht zu der Sitzrichtung ausgerichtet. Unter "zumindest im Wesentlichen senkrecht" soll insbesondere eine Ausrichtung einer Richtung relativ zu einer Bezugsrichtung verstanden werden, wobei die Richtung und die Bezugsrichtung, insbesondere in einer Ebene betrachtet, einen Winkel von 90° einschließen, wobei der Winkel insbesondere eine maximale Abweichung von insbesondere kleiner als 8°, vorteilhaft kleiner als 5° und besonders vorteilhaft kleiner als 2°, aufweist. Vorteilhafterweise bilden die obere Beinstütze und die untere Beinstütze zusammen zumindest einen Teil des Kniegelenks, oder das Kniegelenk, aus. Vorzugsweise entspricht ein Wert des Sitzwinkels einem Wert einer Kniegelenkstellung des Kniegelenks.

Bevorzugt umfasst die tragbare Sitzhaltungshilfevorrichtung zumindest eine Arretierungseinheit, welche dazu vorgesehen ist, das obere Beinteil relativ zum unteren Beinteil und/oder zum Kniegelenk in einem bestimmten Sitzwinkel zu arretieren, und/oder dazu vorgesehen ist, einen kleinsten Sitzwinkel zu definieren. Vorteilhaft ist die Arretierungseinheit dazu vorgesehen, das Kniegelenk in verschiedenen Sitzwinkeln zu arretieren und/oder verschiedene kleinste Sitzwinkel zu definieren, welche vorzugsweise durch die Person auswählbar sind. Es ist denkbar, dass die Verriegelungseinheit dazu vorgesehen ist, eine Vergrößerung des Sitzwinkels im arretierten Zustand zu ermöglichen. Insbesondere ermöglicht sie der Person, aufzustehen, wenn die Arretierungseinheit sich im arretierten Zustand befindet. Bevorzugt ist die Arretierungseinheit dazu vorgesehen, der Person zu ermöglichen, nachdem sie mit der einen bestimmten kleinsten Sitzwinkel definierenden Arretierungseinheit aufgestanden ist, sich mit dem bestimmten definierten kleinsten Sitzwinkel wieder zu setzen. Vorteilhaft weist die Arretierungseinheit zumindest ein Blockierelement auf, welches dazu vorgesehen ist, das Kniegelenk zu blockieren und/oder zu entblocken und/oder den Sitzwinkel zu arretieren und/oder einen kleinsten Sitzwinkel zu definieren. Vorzugsweise ist das Blockierelement als Feder, insbesondere als Gasdruckfeder, ausgebildet. Das Blockierelement ist vorteilhaft mit dem oberen Beinteil, insbesondere der oberen Beinstütze, und mit dem unteren Beinteil, insbesondere der unteren Beinstütze, verbunden. Das Blockierelement ist bevorzugt dazu vorgesehen, eine Bewegung des oberen Beinteils relativ zum unteren Beinteil während eines Niedersitzens und/oder während eines Aufstehens zu dämpfen. Vorteilhaft weist die Arretierungseinheit zumindest ein zum Betätigen des Blockierelements vorgesehenes Betätigungselement auf. Das Betätigungselement ist vorzugsweise dazu vorgesehen, der Person ein Blockieren oder Entblocken des Blockierelements zu ermöglichen. Vorteilhafterweise ist das Betätigungselement ein mechanisches Betätigungselement. Es ist auch denkbar, dass das Betätigungselement ein elektronisches Betätigungselement ist. Des Weiteren ist es denkbar, dass die Arretierungseinheit zumindest eine Steuerungseinheit aufweist, welche dazu vorgesehen ist, entsprechend einem Bedarf nach Blockierung des Blockierelements einen Niedersitzzustand zu erfassen, wenn die Person sich setzt, und/oder einen Aufstehzustand zu erfassen, wenn die Person aufsteht, und/oder das Betätigungselement zu aktivieren.

Ferner wird vorgeschlagen, dass die Beineinheit zumindest eine Beinbeugungsebene definiert, relativ zu welcher die Lageeinheit die Fußeinheit zumindest teilweise zumindest im Wesentlichen parallel translatorisch bewegbar lagert. Es kann vorteilhaft ein Komfort, insbesondere ein Tragekomfort, verbessert werden. Insbesondere kann eine Beweglichkeit bei einer Fortbewegung verbessert werden. Besonders vorteilhaft kann eine Haltung einer Person in einer Sitzposition verbessert werden, insbesondere indem der Person zusätzlicher Bewegungsfreiraum zur Positionierung der Füße relativ zur Beineinheit zur Verfügung gestellt wird. Unter einer "Beinbeugungsebene" soll insbesondere eine Ebene verstanden werden, welche von der Beineinheit, insbesondere von dem oberen Beinteil und dem unteren Beinteil, aufgespannt ist, und zwar insbesondere in einem Zustand, in welchem der Sitzwinkel von einem Winkel von 180° verschieden ist, wie beispielsweise bei einer Sitzhaltung. Alternativ oder zusätzlich könnte die Lagereinheit die Fußeinheit translatorisch zumindest im Wesentlichen senkrecht zur Beinbeugungsebene lagern. Bevorzugt lagert die Lagereinheit zumindest den Teil der Fußeinheit ausschließlich parallel zur Beinbeugungsebene. Die Lagereinheit lagert insbesondere als den Teil der Fußeinheit vorzugsweise den Schuhadapter und/oder die Schuhbindung der Fußeinheit translatorisch zumindest im Wesentlichen parallel zur Beinbeugungsebene.

Weiterhin wird vorgeschlagen, dass die Lagereinheit in einer ersten Lagerstellung eine erste Haupterstreckung und in einer zweiten Lagerstellung eine zweite Haupterstreckung aufweist, welche größer ist als die erste Haupterstreckung. Vorteilhaft kann ein Benutzerkomfort weiter verbessert werden. Insbesondere kann hierdurch eine Bewegungsfreiheit eines Benutzers weiter verbessert werden. Unter einer "Haupterstreckung" eines Objekts soll dabei insbesondere eine längste Kante eines kleinsten geometrischen Quaders, welcher das Objekt gerade noch vollständig umschließt, verstanden werden. Unter einer "Haupterstreckungsrichtung" eines Objekts soll dabei insbesondere eine Richtung verstanden werden, welche parallel zu einer Haupterstreckung des Objekts verläuft. Die Lagereinheit ist insbesondere expandierbar und/oder kontrahierbar ausgebildet. Vorzugsweise ist in der ersten Lagerstellung die Lagereinheit vollständig kontrahiert. Ferner ist in der zweiten Lagerstellung die Lagereinheit vorzugsweise vollständig expandiert. Die zweite Haupterstreckung in der zweiten Lagerstellung der Lagereinheit ist insbesondere zumindest um 5%, vorzugsweise zumindest um 10 %, besonders bevorzug zumindest um 30 % und ganz besonders bevorzugt zumindest um 50 % größer als die erste Haupterstreckung der Lagereinheit in der ersten Lagerstellung. Insbesondere ist zu einer Expansion der Lagereinheit zumindest das weitere Lagerelement entlang dem Lagerelement der Lagereinheit herausziehbar. Insbesondere entspricht die zweite Haupterstreckung der Lagereinheit in der zweiten Lagerstellung zumindest im Wesentlichen der Summe der jeweiligen Haupterstreckungen des Lagerelements, insbesondere des Führungselements des Lagerelements, und des weiteren Lagerelements, insbesondere des weiteren Führungselements des weiteren Lagerelements, der Lagereinheit. Vorzugsweise ist die Lagereinheit in der ersten Lagerstellung zumindest teilweise vorzugsweise zumindest zu einem Großteil und besonders bevorzugt vollständig innerhalb eines Gehäuses angeordnet. Insbesondere weist die Fußeinheit das Gehäuse auf, welches vorteilhaft von der Fußstütze ausgebildet ist.

Es wird ferner vorgeschlagen, dass die Lagereinheit zumindest in einer Expansionsrichtung entgegen der Beineinheit zumindest teilweise, vorzugsweise zumindest zu einem Großteil und besonders bevorzugt vollständig, expandierbar ist. Es kann vorteilhaft ein Komfort weiter verbessert werden. Besonders vorteilhaft kann vermieden werden, dass bei einer Expansion und somit insbesondere einer Positionierung eines Fußes der Person die Beineinheit diese blockiert. Somit kann hierdurch weiter vorteilhaft eine Betriebssicherheit verbessert werden. Insbesondere ist das weitere Lagerelement entlang dem Lagerelement ausziehbar. Unter einer "Expansionsrichtung" soll insbesondere eine Richtung verstanden werden, in welcher die Lagereinheit expandierbar ist, und zwar insbesondere von der ersten Lagerstellung in die zweite Lagerstellung. Die Lagereinheit ist insbesondere zumindest in einer Kontraktionsrichtung in Richtung der Beineinheit zumindest teilweise, vorzugsweise zumindest zu einem Großteil und besonders bevorzugt vollständig, kontrahierbar. Dazu ist das weitere Lagerelement entlang dem Lagerelement einziehbar. Unter einer "Kontraktionsrichtung" soll insbesondere eine Richtung verstanden werden, in welcher die Lagereinheit kontrahierbar ist, und zwar insbesondere von der zweiten Lagerstellung in die erste Lagerstellung. Insbesondere sind die Expansionsrichtung und die Kontraktionsrichtung einander entgegengesetzt, vorzugsweise antiparallel, zueinander ausgerichtet.

Um insbesondere eine Kompaktheit einer Bauform zu verbessern, wird ferner vorgeschlagen, dass die Lagereinheit zumindest einen Teleskopauszug umfasst. Insbesondere bilden das Lagerelement und das weitere Lagerelement den Teleskopauszug aus. Denkbar ist, dass die Lagereinheit mehrere Teleskopauszüge umfasst, welche durch weitere Lagerelemente ausgebildet und ineinander verschachtelt anordenbar sind. Insbesondere bei einem Vorhandensein von mehreren Teleskopauszügen ist denkbar, dass eine zweite Haupterstreckung in der zweiten Lagerstellung der Lagereinheit um zumindest 100 %, vorzugsweise um zumindest 150 % und besonders bevorzugt um zumindest 200 % größer ist als die erste Haupterstreckung der Lagereinheit in der ersten Lagerstellung.

Weiter wird vorgeschlagen, dass die Lagereinheit zumindest ein Rückstellelement aufweist, welches dazu vorgesehen ist, die Fußeinheit in zumindest einer Lagerstellung, insbesondere in einer Lagerstellung, welche von der ersten Lagerstellung verschieden ist, zumindest teilweise mit einer Rückstellkraft zu beaufschlagen. Es kann vorteilhaft ein Komfort weiter verbessert werden. Insbesondere kann ein ungewolltes Auslösen der Lagereinheit vermieden werden. Weiter vorteilhaft kann eine gezielte Rückführung der Lagereinheit erfolgen. Besonders vorteilhaft kann ein physiologischer Bewegungsablauf erzeugt werden. Das Rückstellelement ist insbesondere als ein elastisches Element ausgebildet. Unter einem "elastischen Element" soll insbesondere ein Element verstanden werden, welches wiederholt verformbar ist, ohne dass dadurch das Element mechanisch beschädigt oder zerstört wird, und welches insbesondere nach einer Verformung selbstständig wieder einer Grundform zustrebt. Vorzugsweise ist das elastische Element als eine Feder ausgebildet, wie vorzugsweise eine Spiralfeder. Alternativ oder zusätzlich könnte das elastische Element als ein Gummiband ausgebildet sein. Das Rückstellelement ist insbesondere mit zumindest einem Lagerelement, insbesondere dem weiteren Lagerelement, der Lagereinheit verbunden. Vorzugsweise ist das Rückstellelement zumindest mit dem weiteren Lagerelement verbunden. Alternativ oder zusätzlich kann das Rückstellelement mit einem weiteren Bauteil verbunden sein, wie beispielsweise der Fußeinheit, insbesondere der Fußeinheitsstütze, der Bodenkontakteinheit, der Beineinheit oder dergleichen. Insbesondere bewirkt das Rückstellelement in einer Lagerstellung, welche von der ersten Lagerstellung verschieden ist, wie vorzugsweise der zweiten Lagerstellung, eine Rückstellkraft, welche zumindest quer zur Beinlängsachse, insbesondere der obere und/oder der untere Beinlängsachse, und vorzugsweise zumindest im Wesentlichen parallel zur Beinbeugungsebene gerichtet ist.

Des Weiteren wird vorgeschlagen, dass das Rückstellelement in zumindest einer Lagerstellung, insbesondere in der ersten Lagerstellung, der Lagereinheit zumindest teilweise, vorzugsweise zumindest zu einem Großteil und besonders bevorzugt vollständig verdeckt angeordnet ist, und zwar insbesondere in einer Richtung zumindest im Wesentlichen parallel zur Beineinheit betrachtet. Es kann vorteilhaft eine Betriebssicherheit weiter verbessert werden, da vermieden werden kann, dass Verunreinigungen in die Lagereinheit gelangen und diese blockieren könnten. Weiter vorteilhaft kann ein einheitliches Design erzielt werden. Vorzugsweise ist das Rückstellelement zumindest teilweise, vorzugsweise zumindest zu einem Großteil und besonders bevorzugt innerhalb der Lagereinheit, insbesondere des Lagerelements und/oder des weiteren Lagerelements, angeordnet. Besonders bevorzugt ist das Rückstellelement zwischen dem Lagerelement und dem weiteren Lagerelement angeordnet. Insbesondere weist das weitere Lagerelement eine Ausnehmung auf, in welcher das Rückstellelement zumindest teilweise angeordnet ist. Ferner ist das Rückstellelement zumindest teilweise, vorzugsweise zumindest zu einem Großteil und besonders bevorzugt vollständig von der Fußeinheit, insbesondere der Fußeinheitsstütze, verdeckt.

Es wird weiter vorgeschlagen, dass die Lagereinheit zumindest ein Begrenzungselement, aufweist, welches dazu vorgesehen ist, eine translatorische Bewegung des Teils der Fußeinheit zu begrenzen. Das Begrenzungselement weist insbesondere eine Haupterstreckungsrichtung auf, welche zumindest eine Komponente aufweist, welche im Wesentlichen senkrecht zu einer Führungsrichtung der Lagereinheit ausgerichtet ist. Es kann vorteilhaft eine Fehlfunktion der Lagereinheit, beispielsweise für den Fall, dass das weitere Lagerelement vollständig entlang dem Lagerelement herausgezogen wird, vermieden werden. Insbesondere kann eine besonders bevorzugt Anordnung erzielt werden und/oder ein Bauraum kann verringert und auf weitere Bauteile kann verzichtet werden, wenn vorgeschlagen wird, dass das Begrenzungselement zumindest teilweise einstückig mit zumindest einem Lagerelement, insbesondere dem zuvor genannten Lagerelement und/oder dem zuvor genannten weiteren Lagerelement, der Lagereinheit ausgebildet ist. Alternativ oder zusätzlich kann das Begrenzungselement zumindest teilweise von der Fußeinheit ausgebildet sein, wie beispielsweise von der Fußeinheitsstütze, dem Schuhadapter oder dergleichen.

Um insbesondere einen Komfort weiter zu verbessern, wird vorgeschlagen, dass die Lagereinheit zumindest ein Lagerelement und zumindest ein weiteres Lagerelement, und zwar insbesondere das zuvor genannte Lagerelement und das zuvor genannte weitere Lagerelement, welches zu dem Lagerelement korrespondierend ausgebildet ist, aufweist, deren Haupterstreckungsrichtungen quer zu der Beinlängsachse, insbesondere der obere und/oder der untere Beinlängsachse, und bevorzugt im Wesentlichen parallel zur Beinbeugungsebene ausgerichtet sind.

Es wird ferner vorgeschlagen, dass in zumindest einer Lagerstellung, insbesondere in der zuvor genannten ersten Lagerstellung, das Lagerelement und das weitere Lagerelement zumindest teilweise, vorzugsweise zumindest zu einem Großteil und besonders bevorzugt vollständig, ineinander angeordnet sind. Es kann vorteilhaft eine Sicherheit weiter verbessert werden. Insbesondere ist das Lagerelement zumindest teilweise, vorzugsweise zumindest einem Großteil und besonders bevorzugt vollständig in dem weiteren Lagerelement angeordnet. Vorzugsweise ist das Lagerelement zumindest teilweise von dem weiteren Lagerelement umgriffen, und zwar insbesondere von zumindest zwei Seiten, vorzugsweise von zumindest drei Seiten.

Um insbesondere eine Ergonomie weiter zu verbessern, wird ferner vorgeschlagen, dass die Lagereinheit an einem Endabschnitt der Beineinheit angeordnet ist. Unter einem "Endabschnitt" soll insbesondere ein Abschnitt verstanden werden, welcher sich von einem freien offenen Ende eines Bauteils in Richtung des Bauteils über eine Länge von höchstens 30 %, vorzugsweise höchstens 15 % und besonders bevorzugt höchstens 5 % einer Haupterstreckung des Bauteils erstreckt. Vorzugsweise ist die Lagereinheit an einem Endabschnitt des unteren Beinteils der Beineinheit angeordnet. Insbesondere ist die Lagereinheit an der Fußeinheitsstütze und/oder der Bodenkontakteinheit angeordnet. Die Fußeinheitsstütze und/oder die Bodenkontakteinheit sind insbesondere an dem Endabschnitt der Beineinheit, vorzugsweise des unteren Beinteils der Beineinheit, angeordnet.

Zudem wird ferner vorgeschlagen, dass die Lagereinheit zumindest teilweise, vorzugsweise zumindest zu einem Großteil und besonders bevorzugt vollständig einstückig mit der Fußeinheit ausgebildet ist. Es kann vorteilhaft Aufwand reduziert und auf weitere Bauteile verzichtet werden. Ferner kann vorteilhaft ein einheitliches Erscheinungsbild erzielt werden. Besonders vorteilhaft kann eine Stabilität der Fußeinheit und insbesondere der Lagereinheit verbessert werden. Insbesondere ist zumindest ein weiteres Lagerelement der Lagereinheit zumindest teilweise, vorzugsweise zumindest zu einem Großteil und besonders bevorzugt vollständig einstückig mit dem Schuhadapter ausgebildet. Insbesondere ist das Lagerelement zumindest teilweise, vorzugsweise zumindest zu einem Großteil und besonders bevorzugt vollständig einstückig mit der Fußeinheitsstütze ausgebildet.

Weiterhin wird vorgeschlagen, dass die Fußeinheit zumindest eine zumindest teilweise gummielastische, insbesondere zumindest teilweise aus einem Elastomer ausgebildete, Gelenkeinheit zu einer Anbindung an die Beineinheit umfasst. Insbesondere weist die Gelenkeinheit zumindest einen Gelenkgrundkörper auf, der gummielastisch ausgebildet ist. Darunter, dass die Gelenkeinheit, insbesondere der Gelenkgrundkörper, "gummielastisch" ausgebildet ist, soll insbesondere verstanden werden, dass die Gelenkeinheit, insbesondere der Gelenkgrundkörper, durch während einer Benutzung der tragbaren Sitzhaltungshilfevorrichtung, insbesondere während eines Gehens eines Benutzers mit und/oder während eines Sitzens eines Benutzers auf der tragbaren Sitzhaltungshilfevorrichtung, auf die Gelenkeinheit, insbesondere auf den Gelenkgrundkörper, wirkende Kräfte relativ zu einer Ausgangsform verformbar ist, wobei die Gelenkeinheit, insbesondere der Gelenkgrundkörper, in einem belastungsfreien Zustand, insbesondere eigenständig, in die Ausgangsform zurückkehrt. Insbesondere ist die Gelenkeinheit, insbesondere der Gelenkgrundkörper, unter Einwirkung von während einer Benutzung der tragbaren Sitzhaltungshilfevorrichtung auf die Gelenkeinheit, insbesondere auf den Gelenkgrundkörper, wirkenden Kräften verschieden von plastisch verformbar, insbesondere elastisch verformbar, ausgebildet. Die Gelenkeinheit, insbesondere der Gelenkgrundkörper, ist vorzugsweise mit zumindest einem weiteren Teil der Fußeinheit, insbesondere mit der Fußeinheitsstütze, und/oder mit der Lagereinheit, insbesondere form- und/oder kraftschlüssig, verbunden. Der Gelenkgrundkörper ist bevorzugt als ein zumindest im Wesentlichen L-förmiges bzw. C-förmiges Bauteil ausgebildet. Alternativ sind auch andere, insbesondere einem Fachmann als sinnvoll erscheinende Formgebungen des Gelenkgrundkörpers denkbar. Vorzugsweise erstreckt sich die Beineinheit, insbesondere die untere Beinstütze, durch die Gelenkeinheit, insbesondere durch eine Durchführung in dem Gelenkgrundkörper, hindurch. Insbesondere ist die Beineinheit, insbesondere die untere Beinstütze, mit der Gelenkeinheit, insbesondere mittelbar über zumindest ein Kopplungselement der Fußeinheit, gekoppelt. Der Gelenkgrundkörper ist bevorzugt aus einem Elastomer, insbesondere aus einem Gummi, ausgebildet. Alternativ ist vorstellbar, dass der Gelenkgrundkörper aus einem von einem Elastomer verschiedenen Material ausgebildet ist und insbesondere zumindest ein gummielastisches und/oder stoßdämpfendes Bauteil, wie beispielsweise ein Federelement, einen Pneumatikdämpfer, einen Hydraulikdämpfer, ein Formgedächtnismetall o. dgl., umfasst.

Vorzugsweise ist die Gelenkeinheit, insbesondere der Gelenkgrundkörper, rotatorisch und/oder translatorisch elastisch verformbar ausgebildet. Vorzugsweise ist der Gelenkgrundkörper um eine zumindest im Wesentlichen parallel zu der Kontraktionsrichtung der Lagereinheit verlaufende erste Rotationsachse rotatorisch elastisch verformbar ausgebildet. Insbesondere entspricht eine Rotation eines an die Fußeinheit angebundenen Fußes um die erste Rotationsachse zumindest im Wesentlichen einer Pronation bzw. einer Supination des Fußes. Vorzugsweise ist der Gelenkgrundkörper um eine zumindest im Wesentlichen senkrecht zu der Kontraktionsrichtung der Lagereinheit und zumindest im Wesentlichen senkrecht zu der Beinlängsachse, insbesondere zu der unteren Beinlängsachse, verlaufende zweite Rotationsachse rotatorisch elastisch verformbar ausgebildet. Insbesondere entspricht eine Rotation eines an die Fußeinheit angebundenen Fußes um die zweite Rotationsachse zumindest im Wesentlichen einer Flexion bzw. einer Extension des Fußes. Vorzugsweise ist der Gelenkgrundkörper um eine zumindest im Wesentlichen parallel zu der Beinlängsachse, insbesondere zu der unteren Beinlängsachse, verlaufende dritte Rotationsachse rotatorisch elastisch verformbar ausgebildet. Bevorzugt ist der Gelenkgrundkörper entlang beliebiger Bewegungsachsen, insbesondere zumindest im Wesentlichen senkrecht und/oder zumindest im Wesentlichen parallel zu den Rotationsachsen, translatorisch elastisch verformbar ausgebildet. Insbesondere ist der Gelenkgrundkörper dazu vorgesehen, während einer Benutzung der tragbaren Sitzhaltungshilfevorrichtung, insbesondere während eines Gehens eines Benutzers mit der tragbaren Sitzhaltungshilfevorrichtung, entstehende Schwingungen zu dämpfen, insbesondere durch eine Gummielastizität des Gelenkgrundkörpers. Insbesondere ist die Gelenkeinheit, insbesondere der Gelenkgrundkörper, derart gummielastisch ausgebildet, dass die Gelenkeinheit, insbesondere der Gelenkgrundkörper, unter einer Belastung von höchstens 100 Newton, bevorzugt von höchstens 70 Newton, besonders bevorzugt von höchstens 50 Newton und ganz besonders bevorzugt von höchstens 30 Newton um eine maximale Strecke zwischen 0,1 cm und 7 cm, bevorzugt zwischen 0,1 cm und 5 cm, besonders bevorzugt zwischen 0,1 cm und 3 cm und ganz besonders bevorzugt zwischen 0,1 cm und 2 cm relativ zu der Ausgangsform, insbesondere translatorisch, verformbar ist. Insbesondere ist die Gelenkeinheit, insbesondere der Gelenkgrundkörper, derart gummielastisch ausgebildet, dass die Gelenkeinheit, insbesondere der Gelenkgrundkörper, unter einer Belastung von höchstens 100 Newton, bevorzugt von höchstens 70 Newton, besonders bevorzugt von höchstens 50 Newton und ganz besonders bevorzugt von höchstens 30 Newton um einen maximalen Rotationswinkel zwischen 5° und 60°, bevorzugt zwischen 5° und 45°, besonders bevorzugt zwischen 5° und 30° und ganz besonders bevorzugt zwischen 5° und 20° relativ zu der Ausgangsform rotatorisch verformbar ist. Vorteilhaft kann eine Schwingungsdämpfung während einer Benutzung der tragbaren Sitzhaltungshilfevorrichtung ermöglicht werden. Vorteilhaft kann eine nutzerkomfortable, insbesondere geräuscharm und ermüdungsarm nutzbare, tragbare Sitzhaltungshilfevorrichtung bereitgestellt werden.

Zudem wird vorgeschlagen, dass die Fußeinheit zumindest ein Pufferelement aufweist, das zu einer Aufpralldämpfung zumindest eines mit einer Rückstellkraft beaufschlagten, insbesondere kontrahierenden, Teils der Fußeinheit und/oder der Lagereinheit vorgesehen ist. Das zumindest eine Pufferelement ist vorzugsweise mit der Gelenkeinheit verbunden, insbesondere einstückig mit dem Gelenkgrundkörper ausgebildet. Bevorzugt ist das zumindest eine Pufferelement gummielastisch ausgebildet. Insbesondere ist das zumindest eine Pufferelement aus demselben Material wie der Gelenkgrundkörper, insbesondere aus einem Elastomer, ausgebildet. Alternativ ist vorstellbar, dass das zumindest eine Pufferelement aus einem von einem Material des Gelenkgrundkörpers verschiedenen Material ausgebildet ist. Insbesondere ist das zumindest eine Pufferelement in einem Verbindungsbereich des Gelenkgrundkörpers mit einem Teil der Fußeinheit, insbesondere mit der Fußeinheitsstütze, und/oder mit der Lagereinheit angeordnet. Vorzugsweise dient das zumindest eine Pufferelement als Endanschlag des weiteren Lagerelements in der ersten Lagerstellung der Fußeinheit. Insbesondere ist das zumindest eine Pufferelement dazu vorgesehen, einen Aufprall des weiteren Lagerelements, das von dem Rückstellelement mit einer Rückstellkraft beaufschlagt und insbesondere entlang der Kontraktionsrichtung beschleunigt ist, auf den Gelenkgrundkörper zu dämpfen. Insbesondere weist das zumindest eine Pufferelement stoßdämpfende Eigenschaften auf. Bevorzugt ist das zumindest eine Pufferelement durch eine infolge des Aufpralls des weiteren Lagerelements auf das zumindest eine Pufferelement wirkende Kraft elastisch verformbar ausgebildet. Vorzugsweise weist die Fußeinheit eine Mehrzahl von Pufferelementen auf. Insbesondere weist die Fußeinheit zumindest zwei Pufferelemente auf, die insbesondere entlang einer zumindest im Wesentlichen senkrecht zu der Kontraktionsrichtung verlaufenden Richtung beabstandet voneinander, insbesondere an dem Gelenkgrundkörper, angeordnet sind. Vorteilhaft kann eine tragbare Sitzhaltungshilfevorrichtung mit einer geräuscharmen und abnutzungsarmen Fußeinheit bereitgestellt werden, die einen hohen Tragekomfort ermöglicht.

Des Weiteren wird vorgeschlagen, dass die Gelenkeinheit spielfrei mit zumindest einem Teil der Fußeinheit und/oder der Lagereinheit verbunden, insbesondere verpresst, ist. Vorzugsweise ist die Gelenkeinheit, insbesondere der Gelenkgrundkörper, formschlüssig mit zumindest einem Teil der Fußeinheit und/oder der Lagereinheit verbunden. Bevorzugt ist die Gelenkeinheit, insbesondere der Gelenkgrundkörper, mit dem Lagerelement und/oder mit der Führungsschiene der Lagereinheit spielfrei verbunden, insbesondere verpresst. Alternativ oder zusätzlich ist vorstellbar, dass die Gelenkeinheit, insbesondere der Gelenkgrundkörper, mit zumindest einem Teil der Fußeinheit, insbesondere mit der Fußeinheitsstütze, spielfrei verbunden, insbesondere verpresst, ist. Vorzugsweise sind/ist das Lagerelement und/oder die Führungsschiene in den Gelenkgrundkörper, insbesondere in eine von dem Gelenkgrundkörper begrenzte Aufnahmeöffnung, eingepresst. Vorzugsweise weisen/weist das Lagerelement und/oder die Führungsschiene zumindest abschnittsweise, insbesondere zumindest in einem sich innerhalb des Gelenkgrundkörpers erstreckenden Abschnitts, eine Tannenbaumgeometrie, insbesondere zu einer Realisierung einer Pressverbindung mit dem Gelenkgrundkörper, auf. Vorzugsweise weisen/weist das Lagerelement und/oder die Führungsschiene an dem sich innerhalb des Gelenkgrundkörpers erstreckenden Abschnitts, insbesondere rippenartige, Fortsätze zu einer Realisierung einer spielfreien, insbesondere verdrehsicheren, Verbindung mit dem Gelenkgrundkörper auf.

Insbesondere bilden die Fortsätze die Tannenbaumgeometrie des Lagerelements und/oder der Führungsschiene aus. Vorzugsweise sind die Fortsätze poka-yoke-artig, insbesondere zu einer Realisierung einer vorbestimmten Orientierung der Lagereinheit relativ zu der Gelenkeinheit, insbesondere zu dem Gelenkgrundkörper, angeordnet. Darunter, dass die Fortsätze "poka-yoke-artig" angeordnet sind, soll insbesondere verstanden werden, dass die Fortsätze derart angeordnet sind, dass die Fortsätze eine Verbindung des Lagerelements und/oder der Führungsschiene mit der Gelenkeinheit, insbesondere mit dem Gelenkgrundkörper, in einer einzelnen, insbesondere vorbestimmten, Orientierung relativ zu der Gelenkeinheit, insbesondere zu dem Gelenkgrundkörper, ermöglichen. Vorteilhaft kann eine bauteilarme, einfach montierbare tragbare Sitzhaltungshilfevorrichtung mit einer hohen Betriebs- und/oder Nutzungssicherheit bereitgestellt werden.

Ferner wird vorgeschlagen, dass die Fußeinheit zumindest ein mit der Gelenkeinheit verbundenes Kopplungselement aufweist, durch das sich die Beineinheit zumindest abschnittsweise erstreckt und das von der Gelenkeinheit verschiedene Materialeigenschaften, insbesondere ein härteres Material als die Gelenkeinheit, aufweist. Das Kopplungselement ist vorzugsweise als eine Kopplungshülse ausgebildet. Insbesondere ist das Kopplungselement mit dem Gelenkgrundkörper verbunden. Vorzugsweise ist das Kopplungselement an der Durchführung in dem Gelenkgrundkörper angeordnet, erstreckt sich insbesondere durch die Durchführung in dem Gelenkgrundkörper. Bevorzugt ist das Kopplungselement aus einem härteren Material als der Gelenkgrundkörper ausgebildet. Insbesondere weist das Kopplungselement eine geringere Elastizität als der Gelenkgrundkörper auf. Vorzugsweise ist das Kopplungselement durch während einer Benutzung der tragbaren Sitzhaltungshilfevorrichtung auf das Kopplungselement wirkende Kräfte, insbesondere eine Kraft von höchstens 100 N, zumindest im Wesentlichen unverformbar, insbesondere um eine maximale Strecke von höchstens 1 mm relativ zu einer belastungsfreien Ausgangsform des Kopplungselements verformbar, ausgebildet. Das Kopplungselement ist vorzugsweise aus einem Kunststoff, insbesondere aus einem Duroplasten, ausgebildet. Alternativ ist vorstellbar, dass das Kopplungselement aus einem Metall, aus einem Verbundwerkstoff oder aus einem anderen, einem Fachmann als sinnvoll erscheinenden Material ausgebildet ist. Insbesondere ist das Kopplungselement aus einem von einem Elastomer, insbesondere von einem Gummi, verschiedenen Material ausgebildet. Der, insbesondere aus einem Elastomer ausgebildete, Gelenkgrundkörper ist bevorzugt zumindest stoffschlüssig, insbesondere durch eine Vulkanisation, mit dem Kopplungselement verbunden. Alternativ ist denkbar, dass der Gelenkgrundkörper durch eine Verklebung, durch eine Verrastung, durch eine Verpressung oder durch eine andere, einem Fachmann als sinnvoll erscheinende Verbindungsart mit dem Kopplungselement verbunden ist. Das Kopplungselement ist vorzugsweise zu einer Kopplung mit der Beineinheit, insbesondere mit der unteren Beinstütze, die sich durch das Kopplungselement erstreckt, vorgesehen. Insbesondere weist das Kopplungselement zumindest einen Kopplungsfortsatz, insbesondere einen Kopplungsstift, zu einer Kopplung mit der unteren Beinstütze auf. Der Kopplungsfortsatz erstreckt sich insbesondere in die von dem Kopplungselement bzw. von dem Gelenkgrundkörper begrenzte Durchführung. Der Kopplungsfortsatz ist vorzugsweise dazu vorgesehen, eine Bewegung der Beineinheit, insbesondere der unteren Beinstütze, relativ zu der Fußeinheit, insbesondere zu dem Gelenkgrundkörper, zu ermöglichen. Vorteilhaft kann eine Materialbeanspruchung des Gelenkgrundkörpers durch die Beineinheit gering gehalten werden. Vorteilhaft kann eine und robuste, insbesondere eine lange Lebensdauer aufweisende, tragbare Sitzhaltungshilfevorrichtung mit einem hohen Tragekomfort bereitgestellt werden.

Weiterhin wird vorgeschlagen, dass das Kopplungselement als ein Poka-Yoke-Element ausgebildet ist. Darunter, dass das Kopplungselement als ein "Poka-Yoke-Element" ausgebildet ist, soll insbesondere verstanden werden, dass das Kopplungselement eine Formgebung aufweist, die eine Verbindung des Kopplungselements mit der Gelenkeinheit, insbesondere mit dem Gelenkgrundkörper, in einer einzelnen, insbesondere vorbestimmten, Orientierung relativ zu der Gelenkeinheit, insbesondere zu dem Gelenkgrundkörper, ermöglicht. Insbesondere weist das Kopplungselement entlang einer Umfangsrichtung angeordnete Ausrichtungsfortsätze auf. Vorzugsweise ist eine ungerade Anzahl von Ausrichtungsfortsätzen, insbesondere ein einzelner Ausrichtungsfortsatz, verschieden von übrigen Ausrichtungsfortsätzen ausgebildet. Vorzugsweise weist das Kopplungselement, insbesondere entlang der Umfangsrichtung, eine blumenartige Formgebung auf, wobei insbesondere die Ausrichtungsfortsätze gedachten Blütenblättern einer Blume entsprechen. Vorzugsweise bildet/bilden die, insbesondere blumenartige, Formgebung des Kopplungselements, insbesondere die Ausrichtungsfortsätze, eine Verdrehsicherung des Kopplungselements relativ zu der Gelenkeinheit, insbesondere zu dem Gelenkgrundkörper. Vorzugsweise weisen die Ausrichtungsfortsätze eine organische Form auf. Insbesondere sind die Ausrichtungsfortsätze frei von scharfen Kanten und/oder Ecken, insbesondere abgerundet, ausgebildet. Vorzugsweise ist/sind das Kopplungselement, insbesondere die Ausrichtungsfortsätze, durch die Formgebung dazu vorgesehen, Verspannungen zwischen dem Kopplungselement und der Gelenkeinheit, insbesondere dem Gelenkgrundkörper, gering zu halten. Vorteilhaft kann eine sichere und verschleißarme Verbindung zwischen dem Kopplungselement und der Gelenkeinheit ermöglicht werden. Vorteilhaft kann eine tragbare Sitzhaltungshilfevorrichtung mit einer hohen Betriebs-und/oder Nutzungssicherheit bereitgestellt werden.

Es wird ferner ein Verfahren zum Betrieb einer tragbaren Sitzhaltungshilfevorrichtung vorgeschlagen, bei welchem in zumindest einem Verfahrensschritt eine Fußeinheit, welche zur Anbindung eines Schuhs und/oder eines Fußes einer Person vorgesehen ist, quer zu einer von einer Beineinheit definierten Beinlängsachse, insbesondere einer obere und/oder einer untere Beinlängsachse, translatorisch bewegt wird. Es kann vorteilhaft ein Komfort, insbesondere ein Tragekomfort, und/oder eine Ergonomie verbessert werden.

Es wird ferner vorgeschlagen, dass die Beineinheit zumindest eine Beinbeugungsebene definiert, relativ zu welcher der Schuhadapter und die Schuhbindung zumindest im Wesentlichen senkrecht koppelbar sind. Es kann vorteilhaft ein Komfort, insbesondere ein Tragekomfort, weiter verbessert werden. Insbesondere kann vermieden werden, dass ein Fuß eines Benutzers ungewollt bei einer Bewegung parallel zur Beinbeugungsebene, wie sie beim Gehen oder auch bei einer Bewegung im Sitzen auftreten kann, zu einer ungewollten Entkopplung kommt. Somit kann insbesondere auch eine Betriebssicherheit verbessert werden. Alternativ oder zusätzlich könnten der Schuhadapter und die Schuhbindung zumindest im Wesentlichen parallel zur Beinbeugungsebene koppelbar sein. Bevorzugt sind der Schuhadapter und die Schuhbindung ausschließlich zumindest im Wesentlichen senkrecht zur Beugungsebene koppelbar.

Es wird ferner vorgeschlagen, dass die Schnellkopplung zumindest zu einer kraft-und/oder formschlüssigen Kopplung von Schuhadapter und Schuhbindung vorgesehen ist. Es kann vorteilhaft ein Komfort weiter verbessert werden. Insbesondere durch eine Kombination von Kopplungsmechanismen kann eine stabile und einfach zu handhabende Schnellkopplung bereitgestellt werden. Vorzugsweise umfasst die Schnellkopplung zumindest ein zu einem Kraft- und/oder Formschluss vorgesehenes Schnellkoppelelement. Besonders bevorzugt ist die Schnellkopplung gewindelos und/oder frei von einer Bajonettverbindung ausgebildet.

Es wird ferner vorgeschlagen, dass die Schnellkopplung zumindest ein Magnetelement umfasst, welches zumindest teilweise zur Ausbildung einer Kopplung des Schuhadapters mit der Schuhbindung vorgesehen ist. Es kann vorteilhaft ein Komfort, insbesondere ein Bedienkomfort, weiter verbessert werden. Insbesondere kann hierdurch eine Positionierung des Schuhadapters relativ zur Schuhbindung und/oder deren Kopplung vereinfacht werden. Unter einem "Magnetelement" soll insbesondere ein permanent magnetisches und/oder magnetisierbares Element verstanden werden. Das Magnetelement ist insbesondere zumindest teilweise, vorzugsweise zumindest zu einem Großteil und besonders bevorzugt vollständig, aus einem ferromagnetischen, vorzugsweise weichmagnetischen, und/oder einem hartmagnetischen Material ausgebildet, wie beispielsweise Eisen, Nickel, Kobalt oder dergleichen. Besonders bevorzugt ist das Magnetelement permanentmagnetisch. Insbesondere weist das weitere Schnellkoppelelement das Magnetelement auf. Insbesondere kann das Schnellkoppelelement zumindest teilweise einstückig mit dem Magnetelement ausgebildet sein. Beispielsweise könnte das Rastelement des Schnellkoppelelements einstückig mit dem Magnetelement ausgebildet sein. Ferner weist die Schnellkopplung insbesondere zumindest ein weiteres Magnetelement auf, welches vorzugsweise zu dem Magnetelement korrespondierend ausgebildet ist. Beispielsweise ist das weitere Magnetelement derart ausgestaltet und/oder angeordnet, dass bei einer Kopplung unterschiedliche magnetische Pole des Magnetelements und des weiteren Magnetelement einander zugewandt sind, wodurch sich diese gegenseitig anziehen. Das weitere Magnetelement ist vorzugsweise magnetisierbar. Das weitere Schnellkoppelelement weist insbesondere das weitere Magnetelement auf. Ferner könnte das weitere Schnellkoppelelement zumindest teilweise einstückig mit dem weiteren Magnetelement ausgebildet sein. Vorzugsweise könnte beispielsweise das weitere Rastelement des weiteren Schnellkoppelelements einstückig mit dem weiteren Magnetelement ausgebildet sein. Bevorzugt ist zumindest eines, insbesondere ein einziges, der Magnetelemente aus einem hartmagnetischen Material. Das Magnetelement, insbesondere in Wechselwirkung mit dem weiteren Magnetelement, ist insbesondere dazu vorgesehen, eine magnetische Anziehungskraft zu erzeugen, welche zumindest den Schuhadapter und zumindest die Schuhbindung zumindest quer zur Beinlängsachse, insbesondere der obere und/oder der untere Beinlängsachse, und vorzugsweise quer zur Beinbeugungsebene aufeinander zu bewegt.

Es wird ferner vorgeschlagen, dass das Magnetelement dazu vorgesehen ist, eine Kopplung des Schuhadapters mit der Schuhbindung mittels des Rastelements auszulösen. Es kann vorteilhaft ein Komfort weiter verbessert werden, indem insbesondere ein aufwendiges Auslösen durch die Schnellkopplung, wie beispielsweise durch Bücken, vermieden wird. Insbesondere erzeugt das Magnetelement, insbesondere in Wechselwirkung mit dem weiteren Magnetelement, eine magnetische Anziehungskraft, welche dazu vorgesehen ist, das Rastelement auszulenken und somit insbesondere mit dem weiteren Rastelement zu verrasten. Insbesondere in einem gekoppelten Zustand des Schuhadapters mit der Schuhbindung ist die Kopplung zumindest teilweise durch einen magnetischen Kraftschluss und/oder zumindest teilweise durch einen kraft und/oder formschlüssigen Rastschluss erzeugt.

Es wird ferner vorgeschlagen, dass die Schnellkopplung den Schuhadapter und die Schuhbindung um eine Drehachse quer zur Beinlängsachse, insbesondere der oberen und/oder der unteren Beinlängsachse, lagert. Es kann vorteilhaft ein Komfort, eine Ergonomie und/oder eine Physiologie verbessert werden. Besonders vorteilhaft ermöglicht die Drehachse einen natürlichen Bewegungsablauf des Fußes, welcher so beim Gehen und/oder Sitzen angewinkelt werden kann. Insbesondere bildet das Schnellkoppelelement zumindest eine Achse aus. Das weitere Schnellkoppelelement bildet insbesondere zumindest teilweise eine Achsenaufnahme auf, welche korrespondierend zur Achse ausgebildet ist. Die Achse ist insbesondere in der Achsenaufnahme anordenbar. Die Drehachse ist vorzugsweise von der Haupterstreckungsrichtung der Achse und/oder der Achsenaufnahme definiert.

Es wird ferner vorgeschlagen, dass die tragbare Sitzhaltungshilfevorrichtung zumindest eine Schnelllöseeinheit aufweist, welche dazu vorgesehen ist, den Schuhadapter und die Schuhbindung voneinander werkzeuglos zu lösen. Vorteilhaft können ein Komfort und eine Sicherheit weiter verbessert werden. Unter einer "Schnelllöseeinheit" soll insbesondere eine Einheit verstanden werden, welche dazu vorgesehen ist, eine durch die Schnellkopplung hergestellte Kopplung zumindest zweier Bauteile werkzeuglos, zerstörungsfrei und/oder wiederholbar vorzugsweise einhändig, einbeinig und/oder einfüßig zu entkoppeln, und zwar ganz besonders vorteilhaft mit einer einzigen Handbewegung, Fußbewegung und/oder Beinbewegung einer Person. Vorzugsweise ist die Schnelllöseeinheit dazu vorgesehen, eine Verrastung der Rastelemente aufzuheben und/oder die Magnetelemente voneinander zu beabstanden, so dass deren magnetische Wechselwirkung aufeinander im Wesentlichen verschwindet.

Zudem wird vorgeschlagen, dass die Schnelllöseeinheit zumindest ein Bedienelement zur Entkopplung des Schuhadapters und der Schuhbindung aufweist. Es kann vorteilhaft ein Komfort weiter verbessert werden. Besonders vorteilhaft kann derart eine gewollte Entkopplung gezielt und insbesondere werkzeuglos ausgelöst werden. Vorzugsweise ist das Bedienelement als ein Ziehgriff ausgebildet, welcher insbesondere relativ zum Schuhadapter und/oder zur Schuhbindung bewegbar, insbesondere ausziehbar und/oder eindrückbar ist. Alternativ oder zusätzlich könnte das Bedienelement als ein Bedienhebel und/oder Bedienriegel ausgebildet sein.

Weiterhin wird vorgeschlagen, dass das Bedienelement bei einer Betätigung zumindest mittelbar das Rastelement relativ zu dem Magnetelement verschiebt. Es kann vorteilhaft eine Sicherheit verbessert werden, da nur durch ein gezieltes Auslösen eine Entkopplung initiiert werden kann. Unter "zumindest mittelbar" soll insbesondere mittelbar und/oder unmittelbar verstanden werden. Insbesondere kann die Schnelllöseeinheit zumindest eine Übertragungsmechanik aufweisen, welche dazu vorgesehen ist, das Rastelement relativ zu dem Magnetelement zu verschieben.

Es wird ferner vorgeschlagen, dass das Bedienelement zumindest im Wesentlichen parallel zu der Beinlängsachse, insbesondere der oberen Beinlängsachse und/oder der unteren Beinlängsachse, betätigbar ist. Es kann ein Bedienkomfort weiter verbessert werden, da das Bedienelement besonders einfach von einer Person, welche die Sitzhaltungshilfevorrichtung trägt, erreicht werden kann.

Zudem geht die Erfindung aus von einer Fußeinheit einer erfindungsgemäßen tragbaren Sitzhaltungshilfevorrichtung.

Es wird ferner ein Verfahren zum Betrieb der tragbaren Sitzhaltungshilfevorrichtung vorgeschlagen, bei welchem in zumindest einem Verfahrensschritt der Schuhadapter und die Schuhbindung zumindest quer zu der zumindest einen Beinlängsachse, insbesondere der oberen Beinlängsachse und/oder der unteren Beinlängsachse, der Beineinheit miteinander gekoppelt werden, wobei die Schnellkopplung den Schuhadapter und die Schuhbindung miteinander werkzeuglos koppelt, wobei das zumindest eine Rastelement der Schnellkopplung zumindest teilweise die Kopplung des Schuhadapters mit der Schuhbindung ausbildet. Es kann vorteilhaft ein Komfort, insbesondere ein Tragekomfort, und/oder eine Ergonomie verbessert werden.

Die erfindungsgemäße tragbare Sitzhaltungshilfevorrichtung, die erfindungsgemäße Fußeinheit und/oder das erfindungsgemäße Verfahren zum Betrieb der tragbaren Sitzhaltungshilfevorrichtung sollen/soll hierbei nicht auf die oben beschriebene Anwendung und Ausführungsform beschränkt sein. Insbesondere können/kann die erfindungsgemäße tragbare Sitzhaltungshilfevorrichtung, die erfindungsgemäße Fußeinheit und/oder das erfindungsgemäße Verfahren zum Betrieb der tragbaren Sitzhaltungshilfevorrichtung zu einer Erfüllung einer hierin beschriebenen Funktionsweise eine von einer hierin genannten Anzahl von einzelnen Elementen, Bauteilen und Einheiten sowie Verfahrensschritten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

### Zeichnungen

Weitere Vorteile ergeben sich aus der folgenden Zeichnungsbeschreibung. In den Zeichnungen sind zwei Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine Person mit einer tragbaren Sitzhaltungshilfevorrichtung in einer schematischen Seitenansicht,
- Fig. 2: die tragbare Sitzhaltungshilfevorrichtung in einer schematisch Frontansicht,
- Fig. 3: die tragbare Sitzhaltungshilfevorrichtung in einer schematischen Rückansicht,
- Fig. 4: einen Teil der tragbaren Sitzhaltungshilfevorrichtung mit einer Fußeinheit und einer Lagereinheit in einer schematischen Seitenansicht,
- Fig. 5: einen Teil der tragbaren Sitzhaltungshilfevorrichtung mit der Fußeinheit und der Lagereinheit in einer perspektivischen Ansicht,
- Fig. 6: einen Teil der tragbaren Sitzhaltungshilfevorrichtung mit einem Lagerelement der Lagereinheit in einer perspektivischen Ansicht,
- Fig. 7: einen Teil der tragbaren Sitzhaltungshilfevorrichtung mit einer Schnellkopplung und einem weiteren Lagerelement der Lagereinheit in einer perspektivischen Ansicht,
- Fig. 8: das weitere Lagerelement und einem Teil der Schnellkopplung,
- Fig. 9: einen Teil der Fußeinheit und einem Teil der Schnellkopplung,
- Fig. 10: ein schematisches Ablaufdiagramm eines Verfahrens zum Betrieb der tragbaren Sitzhaltevorrichtung,
- Fig. 11: einen Teil einer alternativen tragbaren Sitzhaltungshilfevorrichtung in einer schematischen Ansicht,
- Fig. 12: einen Teil der alternativen tragbaren Sitzhaltungshilfevorrichtung aus Fig. 11 in einer weiteren schematischen Ansicht und
- Fig. 13: einen Teil der alternativen tragbaren Sitzhaltungshilfevorrichtung aus Fig. 11 in einer schematischen Schnittansicht.

### Beschreibung der Ausführungsbeispiele

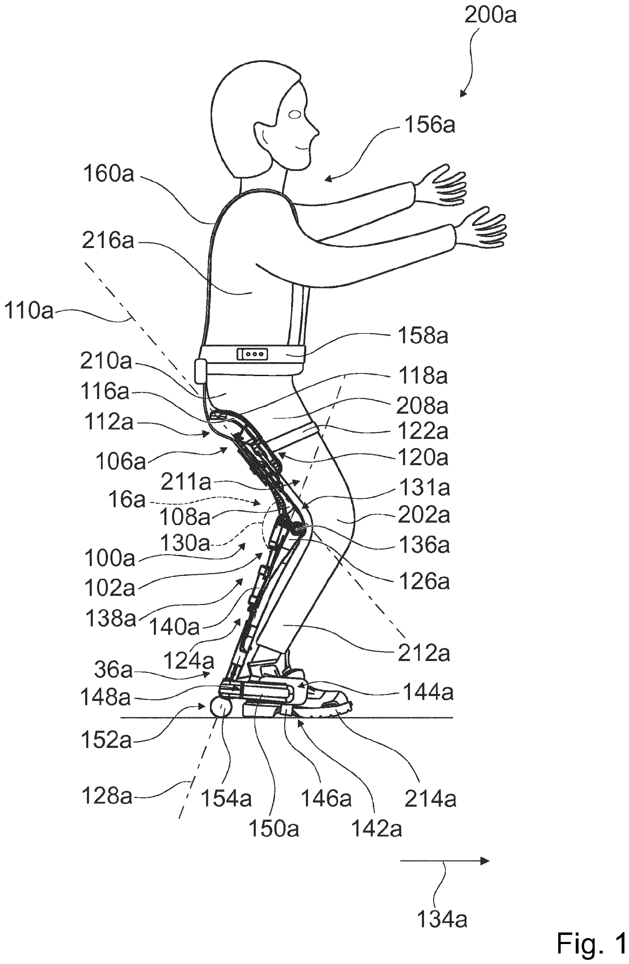

Figur 1 zeigt eine Person 200a, welche eine tragbare Sitzhaltungshilfevorrichtung 100a trägt. Die tragbare Sitzhaltungshilfevorrichtung 100a ist dazu vorgesehen, eine Gewichtskraft der Person 200a in einer Sitzhaltung oder in einer teilweisen Sitzhaltung aufzunehmen. In Figur 1 ist die Person 200a in einer teilweisen Sitzhaltung gezeigt. In der teilweisen Sitzhaltung ist ein Knie 202a der Person 200a teilweise gebeugt. In einer Sitzhaltung ist das Knie 202a stärker gebeugt als in der teilweisen Sitzhaltung. Die tragbare Sitzhaltungshilfevorrichtung 100a ist dazu vorgesehen, es der Person 200a zu ermöglichen, sich in verschiedenen Sitzhaltungen auf die tragbare Sitzhaltungshilfevorrichtung 100a zu setzen und sich in verschiedenen teilweisen Sitzhaltungen auf die tragbare Sitzhaltungshilfevorrichtung 100a teilweise zu setzen. Die tragbare Sitzhaltungshilfevorrichtung 100a ist ferner dazu vorgesehen, der Person 200a zu ermöglichen, zu gehen, während sie die tragbare Sitzhaltungshilfevorrichtung 100a trägt. Die tragbare Sitzhaltungshilfevorrichtung 100a ist außerdem dazu vorgesehen, es der Person 200a zu ermöglichen, zu stehen und/oder aufzustehen und/oder sich zu setzen und/oder zu gehen, während sie die tragbare Sitzhaltungshilfevorrichtung 100a trägt.

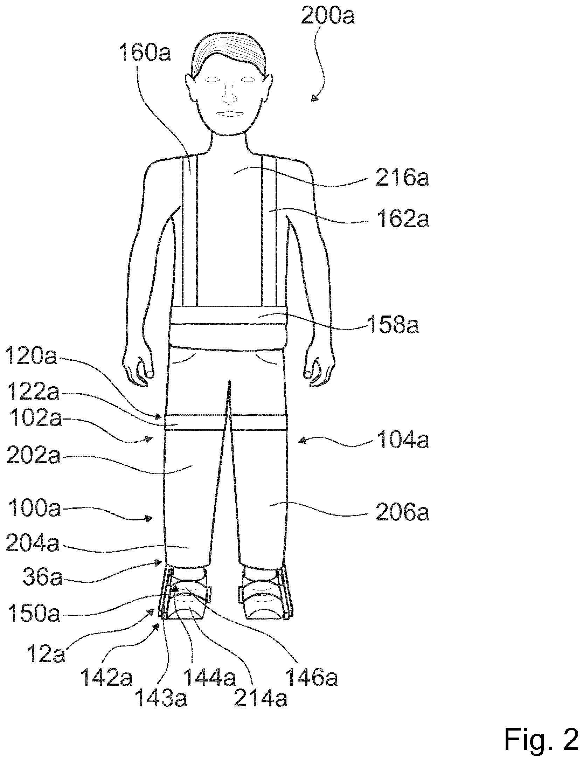

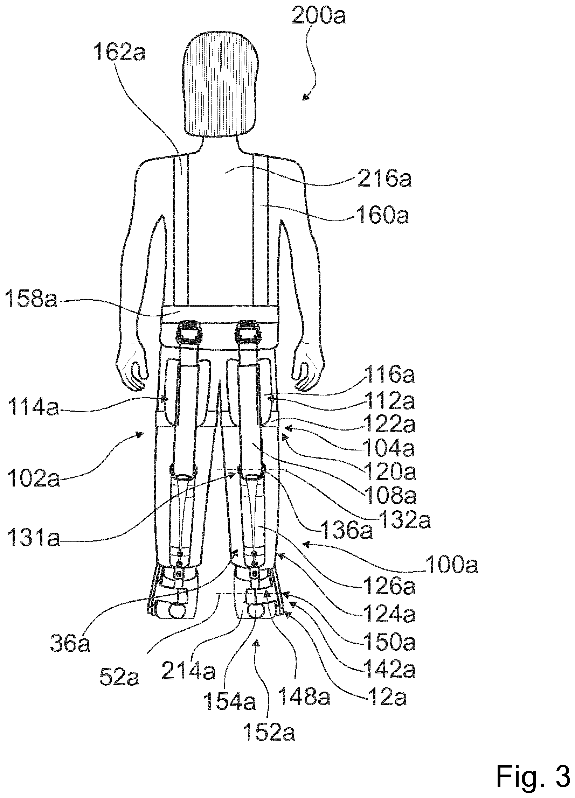

Fig. 2 zeigt die Person 200a, welche die tragbare Sitzhaltungshilfevorrichtung 100a trägt, in einer schematischen Frontansicht. Fig. 3 zeigt die Person 200a, welche die tragbare Sitzhaltungshilfevorrichtung 100a trägt, in einer schematischen Ansicht von hinten. In Fig. 1 bis 3 ist die tragbare Sitzhaltungshilfevorrichtung 100a in einem normalen Tragezustand gezeigt. Der normale Tragezustand umfasst einen Zustand, in welchem die Person 200a auf der tragbaren Sitzhaltungshilfevorrichtung 100a sitzt, einen Zustand, in welchem die Person 200a teilweise sitzt, einen Zustand, in welchem die Person 200a sich hinsetzt, einen Zustand, in welchem die Person 200a steht, sowie einen Zustand, in welchem die Person 200a geht, jeweils während die Person 200a die tragbare Sitzhaltungshilfevorrichtung 100a trägt. In dem abgebildeten Fall trägt die Person 200a die tragbare Sitzhaltungshilfevorrichtung 100a in einem Fabrikgebäude, insbesondere während der Arbeit an einem Fließband. In ähnlicher Weise ist es denkbar, dass die Person 200a die tragbare Sitzhaltungshilfevorrichtung 100a in einem Bürogebäude, in einem Dienstleistungsgebäude, im Freien, bei der Arbeit, zu Hause, während des Arbeitens, während Pausen usw. trägt. Vorteilhafterweise trägt die Person 200a die tragbare Sitzhaltungshilfevorrichtung 100a während einer Aktivität, welche es erfordert, dass die Person 200a immer wieder sich hinsetzt und/oder sich teilweise hinsetzt und/oder aufsteht und/oder steht und/oder geht. Die Person 200a kann sich dann bei Bedarf auf die tragbare Sitzhaltungshilfevorrichtung 100a setzen, während sie die tragbare Sitzhaltungshilfevorrichtung 100a trägt, kann bei Bedarf aufstehen, während sie die tragbare Sitzhaltungshilfevorrichtung 100a trägt, und kann bei Bedarf gehen, während sie die tragbare Sitzhaltungshilfevorrichtung 100a trägt.

Die tragbare Sitzhaltungshilfevorrichtung 100a weist eine Beineinheit 102a auf. Des Weiteren weist die tragbare Sitzhaltungshilfevorrichtung 100a eine zusätzliche Beineinheit 104a auf. Die zusätzliche Beineinheit 104a ist identisch mit der Beineinheit 102a ausgebildet. Aufgrund dessen wird im Folgenden lediglich die Beineinheit 102a ausführlich beschrieben. Die Beschreibung der Beineinheit 102a ist so zu verstehen, dass sie auf die zusätzliche Beineinheit 104a übertragbar ist. Es ist auch denkbar, dass eine zusätzliche Beineinheit spiegelsymmetrisch mit der Beineinheit ausgebildet ist. Es ist insbesondere denkbar, dass eine Beineinheit und eine zusätzliche Beineinheit jeweils als rechte Beineinheit und linke Beineinheit ausgebildet sind oder umgekehrt.

In dem gezeigten Fall trägt die Person 200a die Beineinheit 102a an einem rechten Bein 204a. Die Beineinheit 102a ist auf einer Rückseite 211a des Beins 204a der Person 200a angeordnet. Des Weiteren trägt die Person 200a die zusätzliche Beineinheit 104a an einem linken Bein 206a. Es ist auch denkbar, dass eine Person eine Beineinheit an einem linken Bein trägt und eine zusätzliche Beineinheit an einem rechten Bein trägt. Es ist ferner denkbar, dass eine Person lediglich eine Beineinheit trägt. Dazuhin ist es denkbar, dass eine tragbare Sitzhaltungshilfevorrichtung lediglich eine Beineinheit umfasst. Es ist auch denkbar, dass eine Beineinheit seitlich an einem Bein angeordnet ist und/oder an einer Vorderseite eines Beins und/oder zwischen zwei Beinen einer Person angeordnet ist.

Die Person 200a sitzt oder sitzt teilweise auf der tragbaren Sitzhaltungshilfevorrichtung 100a in Sitzrichtung 134a. Ist die Person 200a nach vorne gewandt, so ist sie in Sitzrichtung 134a gewandt und/oder schaut in Sitzrichtung 134a. Die Sitzrichtung 134a ist parallel zu einem Boden ausgerichtet, oberhalb dessen die Person 200a sitzt oder auf dem die Person 200a geht oder steht.

Die Beineinheit 102a umfasst ein oberes Beinteil 106a. Das obere Beinteil 106a weist eine obere Beinstütze 108a auf. Das obere Beinteil 106a weist eine obere Beinlängsachse 110a auf. Die obere Beinlängsachse 110a ist senkrecht zur Sitzrichtung 134a ausgerichtet. Die obere Beinstütze 108a weist eine Haupterstreckungsrichtung auf, welche parallel zu der oberen Beinlängsachse 110a ausgerichtet ist. Die obere Beinlängsachse 110a ist parallel zu einer Haupterstreckungsrichtung eines Oberschenkels 208a des Beins 204a der Person 200a ausgerichtet, insbesondere wenn die Person 200a sitzt und/oder teilweise sitzt und/oder geht und/oder aufsteht und/oder steht, während sie die tragbare Sitzhaltungshilfevorrichtung 100a trägt.

Das obere Beinteil 106a umfasst eine Sitzeinheit 112a. Die Sitzeinheit 112a ist mit der oberen Beinstütze 108a verbunden. In der teilweisen Sitzhaltung und/oder in der Sitzhaltung sitzt die Person 200a auf der Sitzeinheit 112a. In dem gezeigten Fall sitzt die Person 200a in der teilweisen Sitzhaltung auf der Sitzeinheit 112a und auf einer Sitzeinheit 114a der zusätzlichen Beineinheit 104a. Die Sitzeinheit 112a umfasst ein Sitzelement 116a. Das Sitzelement 116a kontaktiert den Oberschenkel 208a der Person 200a. In der Sitzhaltung und/oder in der teilweisen Sitzhaltung kontaktiert das Sitzelement 116a eine Gesäßbacke 210a der Person 200a. Die Sitzeinheit 112a umfasst eine Sitzfläche 118a. Das Sitzelement 116a weist die Sitzfläche 118a auf. Die Sitzfläche 118a ist dazu vorgesehen, es der Person 200a zu ermöglichen, sich mit dem Oberschenkel 208a und/oder mit der Gesäßbacke 210a auf die Sitzfläche 118a zu setzen. Eine Form der Sitzfläche 118a ist zumindest teilweise an den Oberschenkel 208a und/oder an die Gesäßbacke 210a der Person 200a angepasst. Die Sitzfläche 118a ist gekrümmt. Die Sitzfläche 118a ist konkav gekrümmt und/oder gebogen.

Es ist ebenfalls denkbar, dass eine tragbare Sitzhaltungshilfevorrichtung lediglich eine Sitzeinheit, insbesondere eine gemeinsame Sitzeinheit von zwei Beineinheiten, umfasst. In diesem Fall ist es insbesondere denkbar, dass die Sitzeinheit sattelförmig und/oder wie ein Sattel ausgebildet und/oder insbesondere zwischen den Beinen einer Person angeordnet ist.

Zu einer Anbindung an den Oberschenkel 208a der Person 200a weist das obere Beinteil 106a eine Oberschenkelverbindungseinheit 120a auf. Die Oberschenkelverbindungseinheit 120a ist mit der oberen Beinstütze 108a verbunden. Die Oberschenkelverbindungseinheit 120a ist dazu vorgesehen, das obere Beinteil 106a mit dem Oberschenkel 208a der Person 200a zu verbinden. Die Oberschenkelverbindungseinheit 120a weist ein Oberschenkelband 122a auf. Das Oberschenkelband 122a ist an dem Oberschenkel 208a der Person 200a befestigt.

Die tragbare Sitzhaltungshilfevorrichtung 100a weist ein unteres Beinteil 124a auf. Das untere Beinteil 124a umfasst eine untere Beinstütze 126a. Das untere Beinteil 124a weist eine untere Beinlängsachse 128a auf. Die untere Beinlängsachse 128a ist senkrecht zur Sitzrichtung 134a ausgerichtet. Die untere Beinlängsachse 128a und die obere Beinlängsachse 110a sind in einer gemeinsamen Ebene angeordnet. Die untere Beinstütze 126a weist eine Haupterstreckungsrichtung auf, welche parallel zu der unteren Beinlängsachse 128a ausgerichtet ist. Die untere Beinlängsachse 128a ist, insbesondere wenn die Person 200a, während sie die tragbare Sitzhaltungshilfevorrichtung 100a trägt, sitzt und/oder teilweise sitzt und/oder geht und/oder steht, parallel zu einer Haupterstreckungsrichtung eines Unterschenkels 212a des Beins 204a der Person 200a ausgerichtet.

Das obere Beinteil 106a und das untere Beinteil 124a definieren einen Sitzwinkel 130a. Der Sitzwinkel 130a ist ein zwischen der oberen Beinlängsachse 110a und der unteren Beinlängsachse 128a aufgespannter Winkel. Der Sitzwinkel 130a ist ähnlich oder identisch zu einem Winkel zwischen dem Oberschenkel 208a und dem Unterschenkel 212a der Person 200a. Der einen Wert zwischen 60° und 130°, insbesondere einen Wert von zumindest im Wesentlichen 90°, aufweisende Sitzwinkel 130a entspricht verschiedenen Sitzhaltungen oder zumindest einer Sitzhaltung. Der einen Wert zwischen 130° und 170° aufweisende Sitzwinkel 130a entspricht verschiedenen teilweisen Sitzhaltungen. Wenn die Person 200a steht, während sie die tragbare Sitzhaltungshilfevorrichtung 100a trägt, weist der Sitzwinkel 130a einen Wert zwischen 160° und 180°, insbesondere einen Wert von zumindest im Wesentlichen 180°, auf. Wenn die Person 200a geht, während sie die tragbare Sitzhaltungshilfevorrichtung 100 trägt, kann der Sitzwinkel 130a deutlich von 180° verschieden sein, insbesondere wenn die Person 200a ihr Knie 202a beugt. Der Sitzwinkel 130a und ein analog definierter zusätzlicher Sitzwinkel der zusätzlichen Beineinheit 104a sind in der Sitzhaltung und/oder in der teilweisen Sitzhaltung und/oder beim Stehen vorteilhaft identisch. Es ist jedoch ebenfalls denkbar, dass die Person 200a in einer Sitzhaltung oder teilweisen Sitzhaltung auf der tragbaren Sitzhaltungshilfevorrichtung 100a sitzt, wobei der Sitzwinkel 130a und der zusätzliche Sitzwinkel voneinander verschieden sind, insbesondere um bis zu 5°, um bis zu 10°, um bis zu 15°, um bis zu 20°, um bis zu 30°, um bis zu 40° oder noch mehr voneinander verschieden sind. Wenn die Person 200a geht, während sie die tragbare Sitzhaltungshilfevorrichtung 100a trägt, können der Sitzwinkel 130a und der zusätzliche Sitzwinkel deutlich voneinander verschieden sein, beispielsweise wenn die Person 200a ihre Knie jeweils unterschiedlich beugt.

Die Beineinheit 102a umfasst ein Kniegelenk 131a, welches das obere Beinteil 106a schwenkbar mit dem unteren Beinteil 124a verbindet. Das Kniegelenk 131a verbindet das obere Beinteil 106a mit dem unteren Beinteil 124a um eine Kniegelenkachse 132a schwenkbar. Die Kniegelenkachse 132a ist senkrecht zu der oberen Beinlängsachse 110a ausgerichtet. Die Kniegelenkachse 132a ist senkrecht zu der unteren Beinlängsachse 128a ausgerichtet. Die Kniegelenkachse 132a ist senkrecht zur Sitzrichtung 134a ausgerichtet. Das Kniegelenk 131a ist teilweise einstückig mit der oberen Beinstütze 108a ausgebildet. Das Kniegelenk 131a ist teilweise einstückig mit der unteren Beinstütze 126a ausgebildet. Das Kniegelenk 131a umfasst zumindest ein Lager 136a, welches die obere Beinstütze 108a mit der unteren Beinstütze 126a verbindet.

Die Beineinheit 102a umfasst eine Arretierungseinheit 138a, welche dazu vorgesehen ist, das Kniegelenk 131a zu arretieren. Die Arretierungseinheit 138a ist dazu vorgesehen, den Sitzwinkel 130a auf einen Minimalwert zu begrenzen. Die Arretierungseinheit 138a ist dazu vorgesehen, es der Person 200a zu ermöglichen, den Minimalwert des Sitzwinkels 130a auszuwählen. Befindet sich die Arretierungseinheit 138a in einem arretierten Zustand, kann die Person 200a sich mit dem Minimalwert des Sitzwinkels 130a auf die tragbare Sitzhaltungshilfevorrichtung 100a setzen. Die Arretierungseinheit 138a ist dazu vorgesehen, von der Person 200a betätigt zu werden. Die Arretierungseinheit 138a weist ein Blockierelement 140a auf. Das Blockierelement 140a ist als Feder, insbesondere als Gasdruckfeder, ausgebildet. Das Blockierelement 140a ist dazu vorgesehen, auf unterschiedlichen Längen arretiert zu werden. Das Blockierelement 140a ist mit der oberen Beinstütze 108a verbunden. Das Blockierelement 140a ist mit der unteren Beinstütze 126a verbunden. Das Blockierelement 140a ist dazu vorgesehen, eine Bewegung des oberen Beinteils 106a relativ zum unteren Beinteil 124a zu dämpfen, insbesondere wenn die Person 200a sich hinsetzt.

Die Beineinheit 102a weist eine Bodenkontakteinheit 152a auf. Die Bodenkontakteinheit 152a ist mit einer Fußeinheit 142a verbunden. Die Bodenkontakteinheit 152a ist mit der unteren Beinstütze 126a verbunden. Die Bodenkontakteinheit 152a weist ein Bodenkontaktelement 154a auf. Wenn die Person 200a auf der tragbaren Sitzhaltungshilfevorrichtung 100a sitzt oder teilweise sitzt, ist die Bodenkontakteinheit 152a, insbesondere das Bodenkontaktelement 154a, in Kontakt mit dem Boden. Die Bodenkontakteinheit 152a, insbesondere das Bodenkontaktelement 154a, ist dazu vorgesehen, einen Teil der Gewichtskraft der Person 200a in den Boden abzuleiten. Das Bodenkontaktelement 154a ist abgerundet. Das Bodenkontaktelement 154a ist kugelartig. Das Bodenkontaktelement 154a besteht aus Gummi. Jedoch sind für das Bodenkontaktelement auch andere als die oben genannten Formen und/oder Materialien vorstellbar.

Wenn die Person 200a auf der tragbaren Sitzhaltungshilfevorrichtung 100a sitzt oder teilweise sitzt, wird die Gewichtskraft der Person 200a zumindest teilweise, insbesondere mittelbar oder unmittelbar, von der Sitzeinheit 112a zu der oberen Beinstütze 108a, von der oberen Beinstütze 108a zum Kniegelenk 131a, vom Kniegelenk 131a zu der unteren Beinstütze 126a, von der unteren Beinstütze 126a zu dem Bodenkontaktelement 154a und von dem Bodenkontaktelement 154a zum Boden übertragen.

Insbesondere wird die Gewichtskraft der Person 200a zusätzlich über den Fuß und/oder einen Schuh 214a der Person 200a zum Boden übertragen. Das Bodenkontaktelement 154a ist vorzugsweise auf einer Rückseite des Schuhs 214a der Person 200a angeordnet. Wenn die Person 200a auf der tragbaren Sitzhaltungshilfevorrichtung 100a sitzt oder teilweise sitzt, ist zusätzlich zum Bodenkontaktelement 154a der Fuß und/oder der Schuh 214a der Person 200a in Kontakt mit dem Boden. Bevorzugt ist das Bodenkontaktelement 154a relativ zum Boden kontaktfrei angeordnet, wenn die Person 200a geht und/oder steht, während sie die tragbare Sitzhaltungshilfevorrichtung 100a trägt.

Die tragbare Sitzhaltungshilfevorrichtung 100a weist eine Oberkörpertrageeinheit 156a auf. Die Person 200a trägt die Oberkörpertrageeinheit 156a auf ihrem Oberkörper 216a, wobei der Oberkörper 216a Hüften und/oder eine Taille der Person 200a umfassen kann. Die Oberkörpertrageeinheit 156a weist einen Gürtel 158a auf. Des Weiteren weist die Oberkörpertrageeinheit 156a Hosenträger 160a, 162a auf. Die Beineinheit 102a ist mit der Oberkörpertrageeinheit 156a verbunden. Die zusätzliche Beineinheit 104a ist mit der Oberkörpertrageeinheit 156a verbunden. Es ist denkbar, dass eine Oberkörpertrageeinheit keinen Gürtel und lediglich Hosenträger aufweist oder umgekehrt. Es ist auch denkbar, dass eine tragbare Sitzhaltungshilfevorrichtung lediglich mit den Beinen und/oder den Füßen und/oder den Schuhen einer Person verbunden ist, von der sie getragen wird.

Die Beineinheit 102a umfasst eine Fußeinheit 142a. Die Fußeinheit 142a ist zu einer Anbindung an einen Schuh 214a und/oder einen Fuß der Person 200a vorgesehen. Die Fußeinheit 142a weist eine Schuhbindung 144a zur Anbindung an den Schuh 214a und/oder an den Fuß der Person 200a auf. Der Schuhbindung 144a weist ein Band 146a auf, welches an dem Schuh 214a der Person 200a befestigt ist. Die Fußeinheit 142a ist mit einem Endabschnitt 36a der Beineinheit 102a verbunden. Die Fußeinheit 142a ist relativ zur Beineinheit 102a um die untere Beinlängsachse 128a schwenkbar gelagert. Die Fußeinheit 142a weist eine Fußeinheitsstütze 148a auf. Die Fußeinheitsstütze 148a ist mit dem unteren Beinteil 124a verbunden. Die Fußeinheitsstütze 148a weist einen Bügel 150a auf. Der Bügel 150a ist entlang seiner Haupterstreckungsrichtung um eine Drehachse drehbar gelagert. Der Bügel 150a ist relativ zur Beineinheit 102a drehbar um die Drehachse. Ferner weist die Fußeinheit 142a einen Schuhadapter 143a auf. Der Schuhadapter 143a ist mit der Fußeinheitsstütze 148a zumindest mittelbar verbunden. Der Schuhadapter 143a ist mit der Schuhbindung 144a koppelbar. Mittels des Schuhadapters 143a ist die Schuhbindung 144a mit der Beineinheit 102a koppelbar.

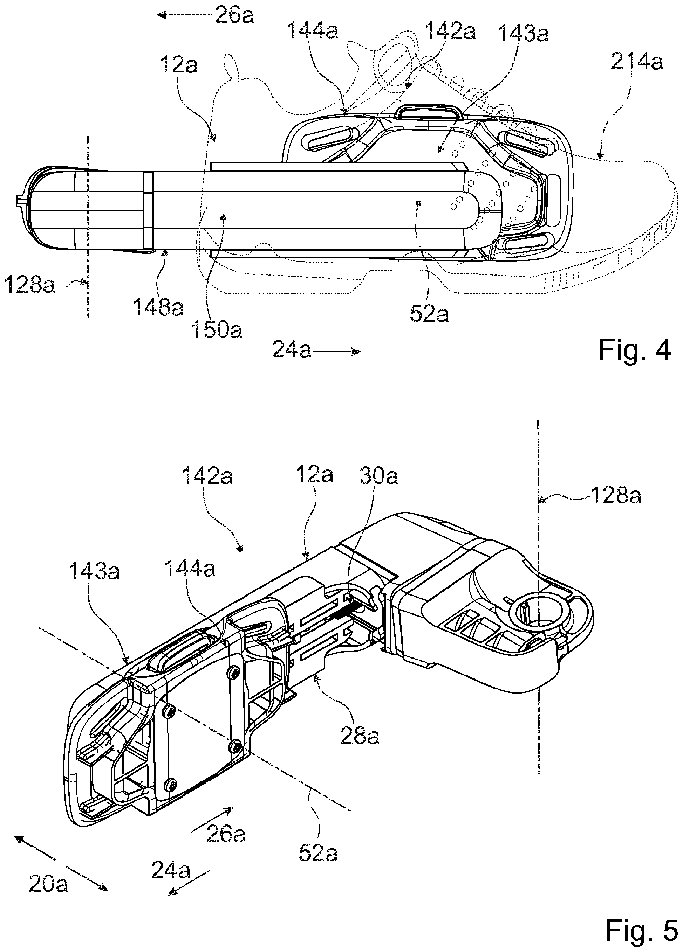

Figuren 4 und 5 zeigen die Fußeinheit 142a in einer schematischen Seitenansicht und einer perspektivischen Innenansicht. Die tragbare Sitzhaltungshilfevorrichtung 100a weist zumindest eine Lagereinheit 12a auf. Die tragbare Sitzhaltungshilfevorrichtung 100a weist pro Beineinheit 102a, 104a eine Lagereinheit 12a auf (vgl. Figuren 2 und 3). Im vorliegenden Fall weist die tragbare Sitzhaltungshilfevorrichtung 100a zwei Lagereinheiten 12a auf. Die Lagereinheiten 12a sind zueinander zumindest im Wesentlichen identisch ausgebildet. Ferner sind die Lagereinheiten 12a spiegelsymmetrisch ausgebildet und/oder angeordnet. Der Übersichtlichkeit halber ist in den Figuren und der Beschreibung nur eine Lagereinheit 12a mit einem Bezugszeichen versehen und näher beschrieben, und zwar insbesondere am Beispiel der Lagereinheit 12a, welche dazu vorgesehen ist, an einem rechten Fuß und/oder Schuh 214a der Person 200a angeordnet zu werden. Die Beschreibung ist auf weitere Lagereinheiten 12a respektive übertragbar. Im Folgenden ist die Lagereinheit 12a näher beschrieben, welche mit der Beineinheit 102a zusammenwirkt.

Die Lagereinheit 12a ist an einem Endabschnitt 36a der Beineinheit 102a angeordnet. Der Endabschnitt 36a erstreckt sich von einem freien offenen Ende der Beineinheit 102a in Richtung der Beineinheit 102a über eine Länge von höchstens 15 % einer Haupterstreckung der Beineinheit 102a. Die Lagereinheit 12a ist im vorliegenden Fall an dem Endabschnitt 36a des unteren Beinteils 124a der Beineinheit 102a angeordnet. Ferner ist die Lagereinheit 12a an der Fußeinheit 142a angeordnet. Ferner ist die Lagereinheit 12a an der Bodenkontakteinheit 152a angeordnet. Die Fußeinheit 142a und/oder die Bodenkontakteinheit 152a ist an dem Endabschnitt 36a der Beineinheit 102a, insbesondere des unteren Beinteils 124a der Beineinheit 102a, angeordnet. Die Fußeinheitsstütze 148a bildet ein Gehäuse aus, in welchem die Lagereinheit 12a zumindest teilweise, vorzugsweise zumindest zu einem Großteil und besonders bevorzugt vollständig angeordnet ist.

Die Lagereinheit 12a lagert zumindest einen Teil der Fußeinheit 142a beweglich. Die Lagereinheit 12a lagert zumindest einen Teil der Fußeinheit 142a zumindest quer zur oberen Beinlängsachse 110a und/oder unteren Beinlängsachse 128a translatorisch bewegbar. Die Lagereinheit 12a lagert zumindest einen Teil der Fußeinheit 142a zumindest im Wesentlichen senkrecht zur oberen Beinlängsachse 110a und/oder unteren Beinlängsachse 128a translatorisch bewegbar. Die translatorische Lagerung entspricht im vorliegenden Fall einer geradlinigen Lagerung. Ferner lagert die Lagereinheit 12a einen Teil der Fußeinheit 142a translatorisch bewegbar zumindest im Wesentlichen parallel zur Beinbeugungsebene 16a. Dabei definiert die Beineinheit 102a in einem angewinkelten Zustand die Beinbeugungsebene 16a. Im vorliegenden Fall lagert die Lagereinheit 12a zumindest den Teil der Fußeinheit 142a ausschließlich parallel zur Beinbeugungsebene 16a. Der Teil der Fußeinheit 142a ist mittels der Lagereinheit 12a relativ zur Beineinheit 102a verschiebbar. Der Teil der Fußeinheit 142a ist im vorliegenden Fall um zumindest 5 cm relativ zur Beineinheit 102a verschiebbar. Im vorliegenden Fall lagert die Lagereinheit 12a als Teil der Fußeinheit 142a den Schuhadapter 143a und/oder die Schuhbindung 144a zueinander. Alternativ oder zusätzlich könnte eine Lagereinheit die Fußeinheit zumindest teilweise translatorisch zumindest im Wesentlichen senkrecht zur Beinbeugungsebene lagern.

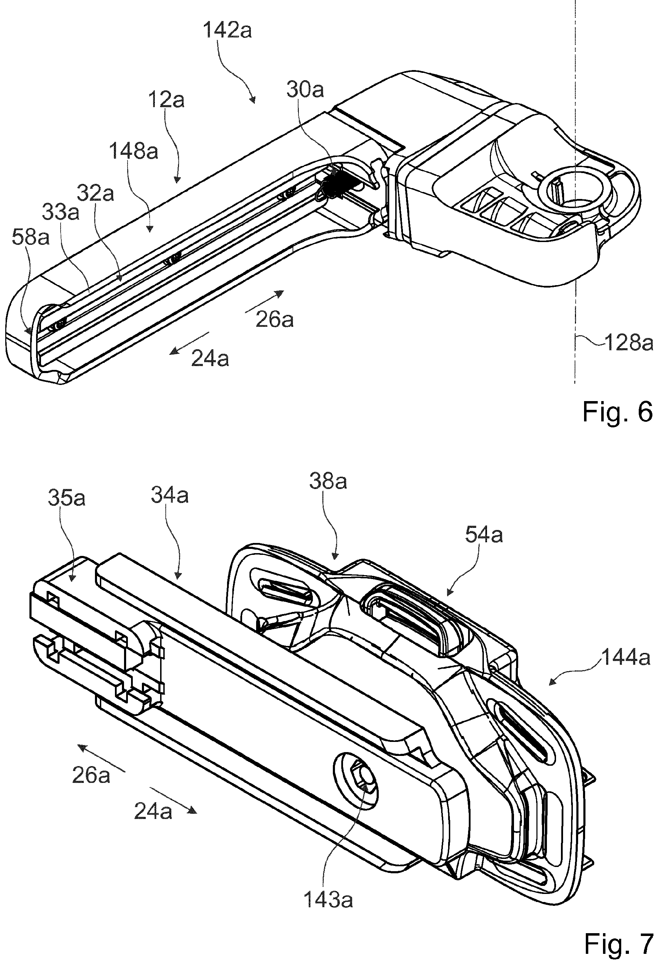

Figuren 6 und 7 zeigen einen Teil der Lagereinheit 12a in schematisch perspektivischen Ansichten. Die Lagereinheit 12a ist im vorliegenden Fall als ein Gleitlager ausgebildet. Die Lagereinheit 12a weist zumindest ein Lagerelement 32a auf. Das Lagerelement 32a ist mit der Fußeinheit 142a verbunden. Das Lagerelement 32a ist in dem Gehäuse, welches von der Fußeinheitsstütze 148a ausgebildet ist angeordnet. Im vorliegenden Fall ist das Lagerelement 32a mit der Fußeinheitsstütze 148a verbunden, insbesondere verschraubt. Alternativ oder zusätzlich kann das Lagerelement zumindest teilweise einstückig mit der Fußeinheit, insbesondere der Fußeinheitsstütze, ausgebildet sein. Ferner kann das Lagerelement auf eine andere, einem Fachmann als vorteilhaft erscheinende Weise mit der Fußeinheit verbunden sein. Das Lagerelement 32a weist eine Führungsschiene 33a auf. Alternativ kann das Lagerelement 32a die Führungsschiene 33a ausbilden.

Zur Ausbildung eines Lagers weist die Lagereinheit 12a zumindest ein weiteres Lagerelement 34a auf. Das weitere Lagerelement 34a ist korrespondierend zu dem Lagerelement 32a ausgebildet. Das weitere Lagerelement 34a ist mit dem Lagerelement 32a verbunden. Das Lagerelement 32a ist relativ zu dem weiteren Lagerelement 34a zumindest verschiebbar. Das weitere Lagerelement 34a weist eine weitere Führungsschiene 35a auf. Die weitere Führungsschiene 35a ist korrespondierend zur Führungsschiene 33a ausgebildet. Das Lagerelement 32a und das weitere Lagerelement 34a führen einander gegenseitig. Alternativ oder zusätzlich könnte eine Lagereinheit als ein Wälzlager ausgebildet sein, wobei insbesondere die Lagereinheit zusätzlich zur Ausbildung des Wälzlagers zumindest ein, vorzugsweise mehrere Wälzelemente umfassen kann, welche zwischen einem Lagerelement und einem weiteren Lagerelement angeordnet sind, um diese aufeinander abwälzend lagern zu können. Beispielsweise könnte ein solches Wälzelement als eine Kugel, eine Rolle oder dergleichen ausgebildet sein.

Die Lagereinheit 12a ist an dem Schuhadapter 143a angeordnet und/oder mit diesem verbunden. Im vorliegenden Fall ist das weitere Lagerelement 34a an dem Schuhadapter 143a angeordnet und/oder mit diesem verbunden. Alternativ könnte eine Lagereinheit an einer anderen Komponente der tragbaren Sitzhaltungshilfevorrichtung 100a angeordnet und/oder mit dieser verbunden sein, wie beispielsweise an/mit einer Beineinheit und/oder einer Bodenkontakteinheit. Insbesondere könnte eine Lagereinheit zwischen dem oberen Beinteil und dem unteren Beinteil angeordnet sein, wie beispielsweise im Bereich eines Kniegelenks. Ferner könnte eine Lagereinheit, insbesondere zumindest eines der Lagerelemente, auch an der Schuhbindung angeordnet sein.

Das Lagerelement 32a und das weitere Lagerelement 34a sind derart angeordnet, dass ihre Haupterstreckungsrichtungen zumindest quer, und zwar insbesondere zumindest im Wesentlichen senkrecht, zu der oberen Beinlängsachse 110a und/oder unteren Beinlängsachse 128a ausgerichtet sind. Ferner sind das Lagerelement 32a und das weitere Lagerelement 34a derart angeordnet, dass ihre Haupterstreckungsrichtungen zumindest im Wesentlichen parallel zur Beinbeugungsebene 16a liegen.

Zumindest in einer Lagerstellung, insbesondere in einer ersten Lagerstellung, sind das Lagerelement 32a und das weitere Lagerelement 34a zumindest teilweise ineinander angeordnet. Im vorliegenden Fall ist weitere Lagerelement 34a zumindest teilweise in dem Lagerelement 32a angeordnet. Das weitere Lagerelement 34a ist zumindest teilweise von dem Lagerelement 32a umgriffen. Das weitere Lagerelement 34a umgreift das weitere Lagerelement 32a von zumindest zwei einander gegenüberliegenden Seiten. Ferner umgreift das weitere Lagerelement 34a zumindest teilweise die Fußeinheitsstütze 148a. Zudem ist das Lagerelement 32a von der Fußeinheitsstütze 148a umgriffen.

Zur Expansion und/oder Kontraktion der Lagereinheit 12a umfasst diese zumindest einen Teleskopauszug 28a. Das Lagerelement 32a und das weitere Lagerelement 34a bilden den Teleskopauszug 28a aus. Alternativ oder zusätzlich könnte die Lagereinheit 12a über mehrere insbesondere stufenartig angeordnete Teleskopauszüge verfügen.

Die Lagereinheit 12a weist eine erste Lagerstellung auf. In der ersten Lagerstellung ist die Lagereinheit 12a kontrahiert. In der ersten Lagerstellung weist die Lagereinheit 12a eine erste Haupterstreckung auf. Zur Expansion ist das weitere Lagerelement 34a der Lagereinheit 12a entlang dem Lagerelement 32a herausziehbar. Die Lagereinheit 12a weist eine zweite Lagerstellung auf. In der zweiten Lagerstellung ist die Lagereinheit 12a vollständig expandiert. In der zweiten Lagerstellung weist die Lagereinheit 12a eine zweite Haupterstreckung auf. Die zweite Haupterstreckung ist größer als die erste Haupterstreckung. Im vorliegenden Fall ist die zweite Haupterstreckung in der zweiten Lagerstellung der Lagereinheit 12a zumindest um 25 % größer als die erste Haupterstreckung der Lagereinheit 12a in der ersten Lagerstellung. Die zweite Haupterstreckung der Lagereinheit 12a in der zweiten Lagerstellung entspricht zumindest im Wesentlichen der Summe der Haupterstreckungen der einzelnen Lagerelemente 32a, 34a der Lagereinheit 12a zusammen. Im vorliegenden Fall ist die Lagereinheit 12a in nur eine einzige Richtung expandierbar. Die Lagereinheit 12a ist zumindest in einer Expansionsrichtung 24a entgegen der Beineinheit 102a zumindest teilweise expandierbar. Im vorliegenden Fall ist die Lagereinheit 12a in nur eine einzige Richtung kontrahierbar. Die Lagereinheit 12a ist zumindest in einer Kontraktionsrichtung 26a in Richtung der Beineinheit 102a zumindest teilweise kontrahierbar. Die Expansionsrichtung 24a und die Kontraktionsrichtung 26a sind einander entgegengesetzt. Die Expansionsrichtung 24a und die Kontraktionsrichtung 26a sind antiparallel zueinander ausgerichtet.

Die Lagereinheit 12a weist zumindest ein Rückstellelement 30a auf. Das Rückstellelement 30a ist dazu vorgesehen, die Fußeinheit 142a in einer Lagerstellung, welche von der ersten Lagerstellung verschieden ist, zumindest teilweise mit einer Rückstellkraft zu beaufschlagen. Die Rückstellkraft zeigt in Richtung der Kontraktionsrichtung 26a. Das Rückstellelement 30a ist als ein elastisches Element ausgebildet. Im vorliegenden Fall ist das Rückstellelement 30a als eine Feder ausgebildet. Alternativ könnte ein Rückstellelement als ein Gummiband oder dergleichen ausgebildet sein.

Das Rückstellelement 30a ist mit dem weiteren Lagerelement 34a der Lagereinheit 12a verbunden. Ferner ist das Rückstellelement 30a mit der Fußeinheitsstütze 148a verbunden. Werden das Lagerelement 32a und das weitere Lagerelement 34a aus der ersten Lagerstellung der Lagereinheit 12a verschoben, so beaufschlagt das Rückstellelement 30a diese mit der Rückstellkraft relativ zueinander. Alternativ könnte ein Rückstellelement mit einem weiteren Bauteil verbunden sein, wie beispielsweise der Bodenkontakteinheit, einer Beineinheit oder dergleichen.

In zumindest einer Lagerstellung ist das Rückstellelement 30a der Lagereinheit 12a zumindest teilweise verdeckt angeordnet. Betrachtet in einer Richtung zumindest im Wesentlichen senkrecht zur Beinbeugungsebene 16a ist das Rückstellelement 30a verdeckt angeordnet. Das Rückstellelement 30a ist zumindest teilweise innerhalb der Lagereinheit 12a angeordnet. Das Rückstellelement 30a ist innerhalb des weiteren Lagerelements 34a angeordnet. Das weitere Lagerelement 34a weist eine Ausnehmung auf. In der Ausnehmung ist das Rückstellelement 30a angeordnet.

Die Lagereinheit 12a weist zumindest ein Begrenzungselement 58a auf. Das Begrenzungselement 58a ist dazu vorgesehen, eine translatorische Bewegung des Teils der Fußeinheit 142a zu begrenzen. Das Begrenzungselement 58a bildet einen Anschlag für die Lagerelemente 32a, 34a aus. Im vorliegenden Fall ist das Begrenzungselement 58a zumindest teilweise von der Fußeinheit 142a gebildet. Das Begrenzungselement 58a ist von der Fußeinheitsstütze 148a gebildet. Alternativ oder zusätzlich könnte ein Begrenzungselement 58a zumindest teilweise einstückig mit zumindest einem Lagerelement 32a, 34a ausgebildet sein.

Der Schuhadapter 143a und die Schuhbindung 144a sind entlang einer Richtung, welche quer zur oberen Beinlängsachse 110a und/oder unteren Beinlängsachse 128a verläuft, miteinander koppelbar, indem diese aufeinander und/oder ineinander entlang dieser Koppelrichtung 20a geführt werden. Ferner sind der Schuhadapter 143a und die Schuhbindung 144a entlang einer Koppelrichtung 20a zumindest im Wesentlichen senkrecht zur Beinbeugungsebene 16a miteinander koppelbar. Insbesondere ist die Koppelrichtung 20a zumindest im Wesentlichen senkrecht zur Expansionsrichtung 24a und/oder Kontraktionsrichtung 26a der Lagereinheit 12a. Im vorliegenden Fall sind der Schuhadapter 143a und die Schuhbindung 144a ausschließlich zumindest im Wesentlichen senkrecht zur Beinbeugungsebene 16a koppelbar. Alternativ oder zusätzlich könnten ein Schuhadapter und eine Schuhbindung zumindest im Wesentlichen parallel zur Beinbeugungsebene koppelbar sein.

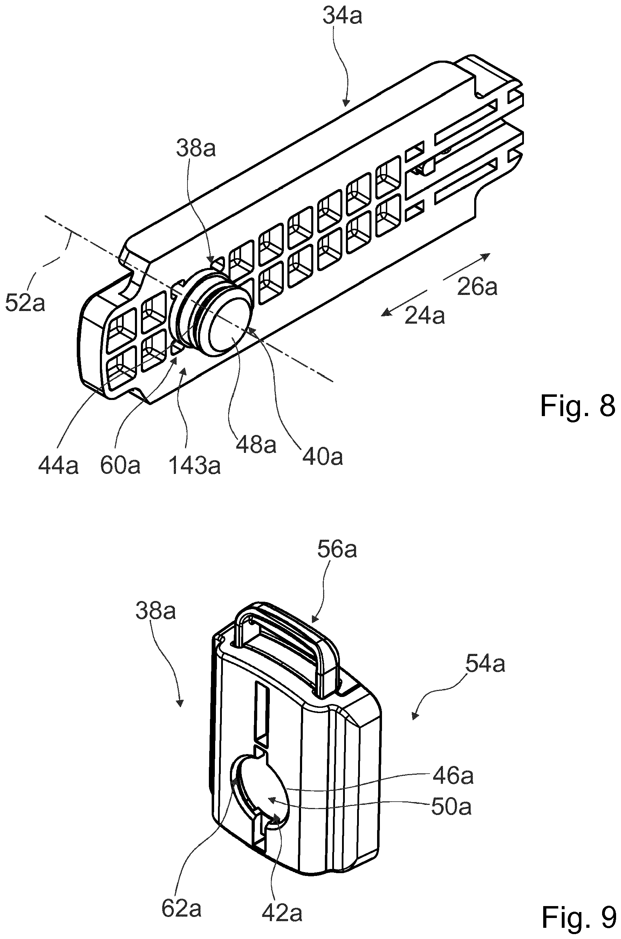

Zur Kopplung des Schuhadapters 143a und der Schuhbindung 144a miteinander weist die tragbare Sitzhaltungshilfevorrichtung 100a zumindest eine Schnellkopplung 38a auf. In Figuren 8 und 9 ist die Schnellkopplung 38a in schematisch perspektivischen Ansichten dargestellt. Mittels der Schnellkopplung 38a sind der Schuhadapter 143a und die Schuhbindung 144a zumindest quer zur oberen Beinlängsachse 110a und/oder unteren Beinlängsachse 128a miteinander koppelbar. Ferner sind der Schuhadapter 143a und die Schuhbindung 144a mittels der Schnellkopplung 38a relativ zur Beinbeugungsebene 16a zumindest im Wesentlichen senkrecht miteinander koppelbar. Die Schnellkopplung 38a ist dazu vorgesehen, den Schuhadapter 143a und die Schuhbindung 144a miteinander werkzeuglos zu koppeln. Die Schnellkopplung 38a ist zu einer wiederholten Kopplung von Schuhadapter 143a und Schuhbindung 144a vorgesehen. Die Schnellkopplung 38a führt eine Kopplung des Schuhadapters 143a mit der Schuhbindung 144a mittels lediglich einer Bein- und/oder Fußbewegung einer Person 200a herbei.

Für die Ausgestaltung der Schnellkopplung 38a sind verschiedene Ausführungsformen einer Kopplung denkbar, wie beispielsweise mittels Klettverschluss, Magnetverschluss, Klipp- und/oder Rastverschluss, Drehverschluss und/oder Bajonettverschluss oder dergleichen. Im vorliegenden Fall ist die Schnellkopplung 38a zumindest teilweise als ein Magnetverschluss ausgebildet. Ferner ist die Schnellkopplung 38a zumindest teilweise als ein Rastverschluss ausgebildet. Die Schnellkopplung 38a ist also als eine Kombination von Magnetverschluss und Rastverschluss ausgebildet.

Zu einer Kopplung weist die Schnellkopplung 38a zumindest ein Schnellkoppelelement 40a auf. Ferner weist die Schnellkopplung 38a zumindest ein weiteres Schnellkoppelelement 42a auf. Das weitere Schnellkoppelelement 42a ist zu dem Schnellkoppelelement 40a korrespondierend ausgebildet.

Die Schnellkopplung 38a ist zumindest teilweise an der Fußeinheit 142a angeordnet. Das Schnellkoppelelement 40a ist an dem Schuhadapter 143a angeordnet. Das weitere Schnellkoppelelement 42a ist an der Schuhbindung 144a angeordnet. Denkbar ist, dass das Schnellkoppelelement zumindest teilweise einstückig mit der Fußeinheit, dem Schuhadapter und/oder der Lagereinheit, insbesondere dem Lagerelement, ausgebildet ist. Ferner ist denkbar, dass das weitere Schnellkoppelelement zumindest teilweise einstückig mit der Fußeinheit, der Schuhbindung und/oder der Lagereinheit, insbesondere dem weiteren Lagerelement, ausgebildet ist.

Die Schnellkopplung 38a ist zumindest zu einer kraft- und/oder formschlüssigen Kopplung von Schuhadapter 143a und Schuhbindung 144a vorgesehen. Die Schnellkopplung 38a weist zumindest ein Rastelement 44a auf. Das Rastelement 44a ist zumindest teilweise zur Ausbildung einer Kopplung des Schuhadapters 143a mit der Schuhbindung 144a vorgesehen. Das Rastelement 44a ist zumindest teilweise elastisch verformbar. Das Rastelement 44a ist dazu vorgesehen, zumindest eine Rastverbindung herzustellen, wie beispielsweise mittels eines Kraft- und/oder Formschlusses. Das Rastelement 44a ist dazu vorgesehen, bei einer Herstellung einer Kopplung zumindest teilweise ausgelenkt und/oder verformt zu werden. Das Rastelement 44a weist einen beispielsweise konusförmigen Vorsprung auf. Ferner weist die Schnellkopplung 38a zumindest ein weiteres Rastelement 46a auf. Das weitere Rastelement 46a ist korrespondierend zu dem Rastelement 44a ausgebildet. Bei einer Kopplung hintergreift das Rastelement 46a das Rastelement 44a, insbesondere den Vorsprung des Rastelements 44a, so dass diese ineinander einrasten. Im vorliegenden Fall weist das Schnellkoppelelement 40a das Rastelement 44a auf. Ferner weist das weitere Schnellkoppelelement 42a das weitere Rastelement 46a auf. Alternativ könnte das Schnellkoppelelement das Rastelement zumindest teilweise einstückig ausbilden und/oder das weitere Schnellkoppelelement das weitere Rastelement zumindest teilweise einstückig ausbilden.

Die Schnellkopplung 38a weist zumindest ein Magnetelement 48a auf. Das Magnetelement 48a ist zumindest teilweise zur Ausbildung einer Kopplung des Schuhadapters 143a mit der Schuhbindung 144a vorgesehen. Das Magnetelement 48a ist permanentmagnetisch ausgebildet. Alternativ könnte das Magnetelement 48a auch magnetisierbar ausgebildet sein. Das Magnetelement 48a ist zumindest teilweise aus einem ferromagnetischen Material ausgebildet. Das Magnetelement 48a ist zumindest teilweise aus einem hartmagnetischen Material ausgebildet. Das Magnetelement 48a ist zumindest teilweise aus Eisen, Nickel, Kobalt oder dergleichen ausgebildet. Ferner weist die Schnellkopplung 38a zumindest ein weiteres Magnetelement 50a auf. Das weitere Magnetelement 50a ist korrespondierend zu dem Magnetelement 48a ausgebildet. Das weitere Magnetelement 50a ist magnetisierbar. Das weitere Magnetelement 50a ist zumindest teilweise aus einem ferromagnetischen Material ausgebildet. Das weitere Magnetelement 50a ist zumindest teilweise aus einem weichmagnetischen Material ausgebildet. Das weitere Magnetelement 50a ist zumindest teilweise aus Eisen, Nickel, Kobalt oder dergleichen ausgebildet. Bei einer Kopplung wechselwirken das Magnetelement 48a und das weitere Magnetelement 50a miteinander, um eine magnetische Anziehungskraft zu erzeugen. Durch eine Wechselwirkung des Magnetelements 48a und des weiteren Magnetelements 50a sind zumindest der Schuhadapter 143a und zumindest die Schuhbindung 144a zumindest quer zur oberen Beinlängsachse 110a und/oder unteren Beinlängsachse 128a aufeinander zu bewegbar. Im vorliegenden Fall weist das Schnellkoppelelement 40a das Magnetelement 48a auf. Ferner weist das weitere Schnellkoppelelement 42a das weitere Magnetelement 50a auf. Alternativ könnte das Schnellkoppelelement das Magnetelement zumindest teilweise einstückig ausbilden und/oder das weitere Schnellkoppelelement das weitere Magnetelement zumindest teilweise einstückig ausbilden. Ferner könnte das Magnetelement zumindest teilweise einstückig mit dem Rastelement und/oder das weitere Magnetelement zumindest teilweise einstückig mit dem weiteren Rastelement ausgebildet sein.

Das Magnetelement 48a löst eine Kopplung des Schuhadapters 143a mit der Schuhbindung 144a mittels des Rastelements 44a aus. Durch die Wechselwirkung zwischen dem Magnetelement 48a und dem weiteren Magnetelement 50a werden das Rastelement 44a und das weitere Rastelement 46a aufeinander zu bewegt. Das Rastelement 44a wird durch das weitere Rastelement 46a ausgelenkt. Das Rastelement 44a verrastet mit dem weiteren Rastelement 46a. In dem gekoppelten Zustand des Schuhadapters 143a mit der Schuhbindung 144a ist die Kopplung zumindest teilweise durch einen kraft- und/oder formschlüssigen Rastschluss erzeugt. Alternativ oder zusätzlich erfolgt die Kopplung zumindest teilweise durch einen magnetischen Kraftschluss durch die Wechselwirkung zwischen dem Magnetelement und dem weiterem Magnetelement.

Ferner lagert die Schnellkopplung 38a in einem gekoppelten Zustand den Schuhadapter 143a und die Schuhbindung 144a um eine Drehachse 52a zumindest quer zur oberen Beinlängsachse 110a und/oder unteren Beinlängsachse 128a. Die Drehachse 52a ist zumindest im Wesentlichen senkrecht zur oberen Beinlängsachse 110a und/oder unteren Beinlängsachse 128a ausgerichtet. Ferner ist die Drehachse 52a zumindest im Wesentlichen senkrecht zur Beinbeugungsebene 16a ausgerichtet. Die Schnellkopplung 38a weist zumindest eine Achse 60a auf. Ferner weist die Schnellkopplung 38a zumindest eine Achsenaufnahme 62a auf. Die Achsenaufnahme 62a ist korrespondierend zur Achse 60a ausgebildet. In einem gekoppelten Zustand ist die Achse 60a zumindest teilweise in der Achsenaufnahme 62a angeordnet. Die Drehachse 52a ist zumindest teilweise durch die Haupterstreckungsrichtung der Achse 60a und/oder der Achsenaufnahme 62a definiert. Im vorliegenden Fall weist das Schnellkoppelelement 40a die Achse 60a auf. Im vorliegenden Fall weist das weitere Schnellkoppelelement 42a die Achsenaufnahme 62a auf.

Die tragbare Sitzhaltungshilfevorrichtung 100a weist zur Entkopplung des Schuhadapters 143a und der Schuhbindung 144a zumindest eine Schnelllöseeinheit 54a auf. Die Schnelllöseeinheit 54a ist zu einer mechanischen Entkopplung der Kopplung vorgesehen. Alternativ könnte eine Entkopplung auch elektronisch ablaufen.

Die tragbare Sitzhaltungshilfevorrichtung 100a weist zumindest eine Schnelllöseeinheit 54a auf, welche dazu vorgesehen ist, den Schuhadapter 143a und die Schuhbindung 144a werkzeuglos voneinander zu lösen. Die Schnelllöseeinheit 54a ist dazu vorgesehen, eine durch die Schnellkopplung 38a hergestellte Kopplung des Schuhadapters 143a und der Schuhbindung 144a werkzeuglos, zerstörungsfrei und/oder wiederholbar zu lösen. Ferner ist die Kopplung einhändig, einbeinig und/oder einfüßig entkoppelbar, und zwar vorteilhaft mit einer einzigen Handbewegung, Fußbewegung und/oder Beinbewegung der Person 200a. Die Schnelllöseeinheit 54a ist dazu vorgesehen, eine Verrastung der Rastelemente 44a, 46a aufzuheben und/oder die Magnetelemente 48a, 50a voneinander zu beabstanden, so dass deren magnetischen Anziehungskräfte aufeinander im Wesentlichen verschwinden.

Die Schnelllöseeinheit 54a weist zur Entkopplung zumindest ein Bedienelement 56a auf. Das Bedienelement 56a ist im vorliegenden Fall als ein Bügel ausgebildet. Alternativ könnte ein Bedienelement 56a auch als ein Bedienhebel, Bedienknopf und/oder Bedienriegel ausgebildet sein. Zur Betätigung der Schnelllöseeinheit 54a ist das Bedienelement 56a relativ zum Schuhadapter 143a und/oder zur Schuhbindung 144a bewegbar. Das Bedienelement 56a ist zumindest im Wesentlichen parallel zur oberen Beinlängsachse 110a und/oder unteren Beinlängsachse 128a betätigbar. Das Bedienelement 56a ist ausziehbar.

Bei einer Betätigung verschiebt das Bedienelement 56a zumindest mittelbar das Rastelement 44a relativ zu dem Magnetelement 48a. Im vorliegenden Fall verschiebt das Bedienelement 56a unmittelbar das Rastelement 44a. Alternativ kann eine Schnelllöseeinheit zumindest eine Übertragungsmechanik aufweisen, welche dazu vorgesehen ist, das Rastelement relativ zu dem Magnetelement bei einer Betätigung des Bedienelements zu verschieben. Durch die Betätigung wird das weitere Rastelement 46a verschoben und/oder verformt, so dass das Rastelement 44a und das weitere Rastelement 46a voneinander entrasten. Ferner ist das Magnetelement 48a relativ zu dem weiteren Magnetelement 50a mittels des Bedienelements 56a verschiebbar, so dass eine magnetische Wechselwirkung der Magnetelemente 48a, 50a unterbrochen ist.

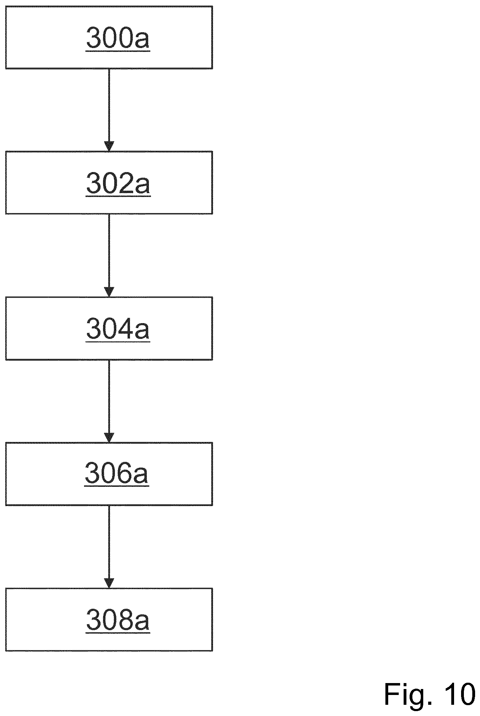

Figur 10 zeigt einen schematischen Ablaufplan eines Verfahrens zum Betrieb der tragbaren Sitzhaltungshilfevorrichtung 100a.

Das Verfahren umfasst zumindest einen Verfahrensschritt 300a. In dem Verfahrensschritt 300a wird ein Fuß und/oder ein Schuh 214a einer Person 200a mit der Schuhbindung 144a verbunden.

Das Verfahren umfasst zumindest einen weiteren Verfahrensschritt 302a. In dem weiteren Verfahrensschritt 302a wird der Schuhadapter 143a und die Schuhbindung 144a zumindest quer zur oberen Beinlängsachse 110a und/oder unteren Beinlängsachse 128a der Beineinheit 102a miteinander gekoppelt, wobei die Schnellkopplung 38a den Schuhadapter 143a und die Schuhbindung 144a miteinander werkzeuglos koppelt, wobei das zumindest eine Rastelement 44a der Schnellkopplung 38a zumindest teilweise die Kopplung des Schuhadapters 143a mit der Schuhbindung 144a ausbildet. Dazu wird die Schuhbindung 144a von der die Schuhbindung 144a tragenden Person 200a mit dem Fuß in einen Nahbereich des Schuhadapters 143a bewegt. Das Magnetelement 48a wechselwirkt mit dem weiteren Magnetelement 50a. Eine resultierende magnetische Kraft bewegt die Schuhbindung 144a und den Schuhadapter 143a aufeinander zu. Eine Kopplung des Schuhadapters 143a und der Schuhbindung 144a wird eingeleitet.

Das Verfahren umfasst zumindest einen weiteren Verfahrensschritt 304a. In dem weiteren Verfahrensschritt 304a wird durch die von den Magnetelement 48a, 50a hervorgerufene Anziehungskraft das Rastelement 44a mit dem weiteren Rastelement 46a verrastet. Es wird eine Kopplung der Schuhbindung 144a mit dem Schuhadapter 143a hervorgerufen. Die Kopplung erfolgt zumindest teilweise, insbesondere zumindest zu einem Großteil und besonders bevorzugt vollständig über eine Verrastung der Rastelemente 44a, 46a. Alternativ oder zusätzlich kann die Kopplung zumindest teilweise magnetisch erfolgen.

Das Verfahren umfasst einen weiteren Verfahrensschritt 306a. In dem weiteren Verfahrensschritt 306a kann sich eine Person 200a, welche die tragbare Sitzhaltungshilfevorrichtung 100a trägt, frei fortbewegen. Zumindest der Teil der Fußeinheit 142a, welcher zur Anbindung eines Schuhs 214a und/oder eines Fußes einer Person 200a vorgesehen ist, und zwar insbesondere der Schuhadapter 143a und die Schuhbindung 144a, wird quer zu der oberen Beinlängsachse 110a und/oder unteren Beinlängsachse 128a translatorisch bewegt. Die Bewegung wird von der Lagereinheit 12a ermöglicht. Alternativ oder zusätzlich kann eine Bewegung eines Teils der Fußeinheit 142a quer oberen Beinlängsachse und/oder unteren Beinlängsachse auch in einem sitzenden Zustand erfolgen, beispielsweise um eine Sitzhaltung zu verändern.

Das Verfahren umfasst zumindest einen weiteren Verfahrensschritt 308a. In dem weiteren Verfahrensschritt 308a wird eine Kopplung zwischen Schuhadapter 143a und Schuhbindung 144a aufgehoben. Die Schnelllöseeinheit 54a löst die Kopplung. Die Person 200a betätigt das Bedienelement 56a. Das Bedienelement 56a wird durch einhändiges Herausziehen betätigt. Das Bedienelement 56a löst eine Entkopplung aus. Das Bedienelement 56a verschiebt die Rastelemente 44a, 46a zueinander. Das Bedienelement 56a entrastet die Rastelemente 44a, 46a voneinander. Ferner verschiebt das Bedienelement 56a die Magnetelemente 48a, 50a zueinander, so dass deren Wechselwirkung zumindest im Wesentlichen vernachlässigbar wird. Der Schuhadapter 143a und die Schuhbindung 144a werden voneinander entkoppelt.

Hinsichtlich weiterer Verfahrensschritte des Verfahrens zum Betrieb der tragbaren Sitzhaltungshilfevorrichtung 100a darf auf die vorhergehende Beschreibung der tragbaren Sitzhaltungshilfevorrichtung 100a verwiesen werden, da diese Beschreibung analog auch auf das Verfahren zu lesen ist und somit alle Merkmale hinsichtlich der tragbaren Sitzhaltungshilfevorrichtung 100a auch in Bezug auf das Verfahren zum Betrieb der tragbaren Sitzhaltungshilfevorrichtung 100a als offenbart gelten.

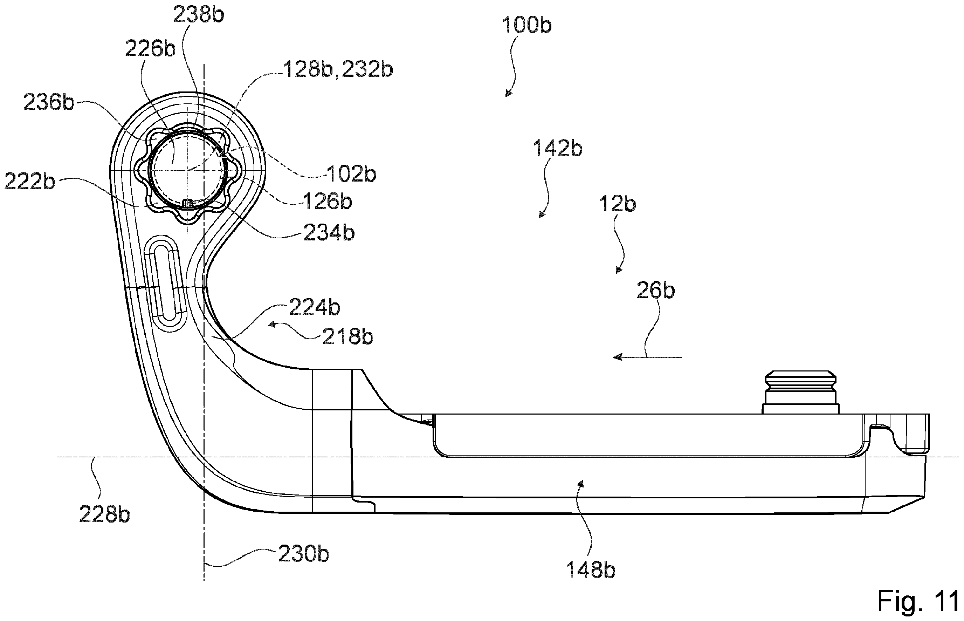

In den Figuren 11 bis 13 ist ein weiteres Ausführungsbeispiel der Erfindung gezeigt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung des anderen Ausführungsbeispiels, insbesondere der Figuren 1 bis 10, verwiesen werden kann. Zur Unterscheidung der Ausführungsbeispiele ist der Buchstabe a den Bezugszeichen des Ausführungsbeispiels in den Figuren 1 bis 10 nachgestellt. In dem Ausführungsbeispiel der Figuren 11 bis 13 ist der Buchstabe a durch den Buchstaben b ersetzt.

Figur 11 zeigt einen Teil einer alternativen tragbaren Sitzhaltungshilfevorrichtung 100b in einer schematischen Ansicht. Dargestellt sind eine Fußeinheit 142b, eine Lagereinheit 12b und ein Teil einer Beineinheit 102b der tragbaren Sitzhaltungshilfevorrichtung 100b. Die Fußeinheit 142b umfasst zumindest eine zumindest teilweise gummielastische, insbesondere zumindest teilweise aus einem Elastomer ausgebildete, Gelenkeinheit 218b zu einer Anbindung an die Beineinheit 102b. Die tragbare Sitzhaltungshilfevorrichtung 100b umfasst insbesondere eine weitere Fußeinheit, eine weitere Lagereinheit, eine weitere Gelenkeinheit und eine weitere Beineinheit, die der Übersichtlichkeit halber hier nicht dargestellt sind. Die folgende Beschreibung der Fußeinheit 142b, der Lagereinheit 12b, der Gelenkeinheit 218b und der Beineinheit 102b ist insbesondere analog auf die weitere Fußeinheit, die weitere Lagereinheit, die weitere Gelenkeinheit und die weitere Beineinheit übertragbar. Die Gelenkeinheit 218b weist zumindest einen Gelenkgrundkörper 224b auf, der gummielastisch ausgebildet ist. Die Gelenkeinheit 218b, insbesondere der Gelenkgrundkörper 224b, ist unter Einwirkung von während einer Benutzung der tragbaren Sitzhaltungshilfevorrichtung 100b auf die Gelenkeinheit 218b, insbesondere auf den Gelenkgrundkörper 224b, wirkenden Kräften verschieden von plastisch verformbar, insbesondere elastisch verformbar, ausgebildet. Die Gelenkeinheit 218b, insbesondere der Gelenkgrundkörper 224b, ist mit zumindest einem weiteren Teil der Fußeinheit 142b, insbesondere mit einer Fußeinheitsstütze 148b der Fußeinheit 142b, und/oder mit der Lagereinheit 12b, insbesondere form- und/oder kraftschlüssig, verbunden. Der Gelenkgrundkörper 224b ist als ein zumindest im Wesentlichen L-förmiges bzw. C-förmiges Bauteil ausgebildet. Alternativ sind auch andere, insbesondere einem Fachmann als sinnvoll erscheinende Formgebungen des Gelenkgrundkörpers 224b denkbar. Die Beineinheit 102b, insbesondere eine untere Beinstütze 126b der Beineinheit 102b, erstreckt sich durch die Gelenkeinheit 218b, insbesondere durch eine Durchführung 226b in dem Gelenkgrundkörper 224b, hindurch. Die Beineinheit 102b, insbesondere die untere Beinstütze 126b, ist, insbesondere mittelbar über zumindest ein Kopplungselement 222b der Fußeinheit 142b, mit der Gelenkeinheit 218b gekoppelt. Der Gelenkgrundkörper 224b ist aus einem Elastomer, insbesondere aus einem Gummi, ausgebildet. Alternativ ist vorstellbar, dass der Gelenkgrundkörper 224b aus einem von einem Elastomer verschiedenen Material ausgebildet ist und insbesondere zumindest ein gummielastisches und/oder stoßdämpfendes Bauteil, wie beispielsweise ein Federelement, einen Pneumatikdämpfer, einen Hydraulikdämpfer, ein Formgedächtnismetall o. dgl., umfasst.

Die Gelenkeinheit 218b, insbesondere der Gelenkgrundkörper 224b, ist rotatorisch und/oder translatorisch elastisch verformbar ausgebildet. Im vorliegenden Ausführungsbeispiel ist die Gelenkeinheit 218b, insbesondere der Gelenkgrundkörper 224b, beispielhaft rotatorisch und translatorisch elastisch verformbar ausgebildet. Der Gelenkgrundkörper 224b ist um eine zumindest im Wesentlichen parallel zu einer Kontraktionsrichtung 26b der Lagereinheit 12b verlaufende erste Rotationsachse 228b rotatorisch elastisch verformbar ausgebildet. Eine Rotation eines an die Fußeinheit 142b angebundenen Fußes um die erste Rotationsachse 228b entspricht zumindest im Wesentlichen einer Pronation bzw. einer Supination des Fußes (hier nicht weiter dargestellt). Der Gelenkgrundkörper 224b ist um eine zumindest im Wesentlichen senkrecht zu der Kontraktionsrichtung 26b der Lagereinheit 12b und zumindest im Wesentlichen senkrecht zu einer Beinlängsachse, insbesondere zu einer unteren Beinlängsachse 128b, verlaufende zweite Rotationsachse 230b rotatorisch elastisch verformbar ausgebildet. Eine Rotation eines an die Fußeinheit 142b angebundenen Fußes um die zweite Rotationsachse 230b entspricht zumindest im Wesentlichen einer Flexion bzw. einer Extension des Fußes. Der Gelenkgrundkörper 224b ist um eine zumindest im Wesentlichen parallel zu der Beinlängsachse, insbesondere zu der unteren Beinlängsachse 128b, verlaufende dritte Rotationsachse 232b rotatorisch elastisch verformbar ausgebildet. Der Gelenkgrundkörper 224b ist entlang beliebiger Bewegungsachsen, insbesondere zumindest im Wesentlichen senkrecht und/oder zumindest im Wesentlichen parallel zu den Rotationsachsen 228b, 230b, 232b, translatorisch elastisch verformbar ausgebildet. Der Gelenkgrundkörper 224b ist dazu vorgesehen, während einer Benutzung der tragbaren Sitzhaltungshilfevorrichtung 100b, insbesondere während eines Gehens eines Benutzers mit der tragbaren Sitzhaltungshilfevorrichtung 100b, entstehende Schwingungen zu dämpfen, insbesondere durch eine Gummielastizität des Gelenkgrundkörpers 224b. insbesondere ist die Gelenkeinheit 218b, insbesondere der Gelenkgrundkörper 224b, derart gummielastisch ausgebildet, dass die Gelenkeinheit 218b, insbesondere der Gelenkgrundkörper 224b, unter einer Belastung von höchstens 100 Newton, bevorzugt von höchstens 70 Newton, besonders bevorzugt von höchstens 50 Newton und ganz besonders bevorzugt von höchstens 30 Newton um eine maximale Strecke zwischen 0,1 cm und 7 cm, bevorzugt zwischen 0,1 cm und 5 cm, besonders bevorzugt zwischen 0,1 cm und 3 cm und ganz besonders bevorzugt zwischen 0,1 cm und 2 cm relativ zu einer Ausgangsform in einem belastungsfreien Zustand der Gelenkeinheit 218b, insbesondere translatorisch, verformbar ist. Insbesondere ist die Gelenkeinheit 218b, insbesondere der Gelenkgrundkörper 224b, derart gummielastisch ausgebildet, dass die Gelenkeinheit 218b, insbesondere der Gelenkgrundkörper 224b, unter einer Belastung von höchstens 100 Newton, bevorzugt von höchstens 70 Newton, besonders bevorzugt von höchstens 50 Newton und ganz besonders bevorzugt von höchstens 30 Newton um einen maximalen Rotationswinkel zwischen 5° und 60°, bevorzugt zwischen 5° und 45°, besonders bevorzugt zwischen 5° und 30° und ganz besonders bevorzugt zwischen 5° und 20° relativ zu der Ausgangsform rotatorisch verformbar ist.

Die Fußeinheit 142b weist zumindest ein mit der Gelenkeinheit 218b verbundenes Kopplungselement 222b auf, durch das sich die Beineinheit 102b zumindest abschnittsweise erstreckt und das von der Gelenkeinheit 218b verschiedene Materialeigenschaften, insbesondere ein härteres Material als die Gelenkeinheit 218b, aufweist. Das Kopplungselement 222b ist als eine Kopplungshülse ausgebildet. Das Kopplungselement 222b ist mit dem Gelenkgrundkörper 224b verbunden. Das Kopplungselement 222b ist an der Durchführung 226b in dem Gelenkgrundkörper 224b angeordnet, erstreckt sich insbesondere durch die Durchführung 226b in dem Gelenkgrundkörper 224b. Das Kopplungselement 222b ist aus einem härteren Material als der Gelenkgrundkörper 224b ausgebildet. Das Kopplungselement 222b weist eine geringere Elastizität als der Gelenkgrundkörper 224b auf. Das Kopplungselement 222b ist durch während einer Benutzung der tragbaren Sitzhaltungshilfevorrichtung 100b auf das Kopplungselement 222b wirkende Kräfte, insbesondere eine Kraft von höchstens 100 N, zumindest im Wesentlichen unverformbar, insbesondere um eine maximale Strecke von höchstens 1 mm relativ zu einer belastungsfreien Ausgangsform des Kopplungselements 222b verformbar, ausgebildet. Das Kopplungselement 222b ist aus einem Kunststoff, insbesondere aus einem Duroplasten, ausgebildet. Alternativ ist vorstellbar, dass das Kopplungselement 222b aus einem Metall, aus einem Verbundwerkstoff oder aus einem anderen, einem Fachmann als sinnvoll erscheinenden Material ausgebildet ist. Das Kopplungselement 222b ist aus einem von einem Elastomer, insbesondere von einem Gummi, verschiedenen Material ausgebildet. Der, insbesondere aus einem Elastomer ausgebildete, Gelenkgrundkörper 224b ist zumindest stoffschlüssig, insbesondere durch eine Vulkanisation, mit dem Kopplungselement 222b verbunden. Alternativ ist denkbar, dass der Gelenkgrundkörper 224b durch eine Verklebung, durch eine Verrastung, durch eine Verpressung oder durch eine andere, einem Fachmann als sinnvoll erscheinende Verbindungsart mit dem Kopplungselement 222b verbunden ist. Das Kopplungselement 222b ist zu einer Kopplung mit der Beineinheit 102b, insbesondere mit der unteren Beinstütze 126b, die sich durch das Kopplungselement 222b erstreckt, vorgesehen. Das Kopplungselement 222b weist zumindest einen Kopplungsfortsatz 234b, insbesondere einen Kopplungsstift, zu einer Kopplung mit der unteren Beinstütze 126b auf. Der Kopplungsfortsatz 234b erstreckt sich in die von dem Kopplungselement 222b bzw. von dem Gelenkgrundkörper 224b begrenzte Durchführung 226b. Der Kopplungsfortsatz 234b ist dazu vorgesehen, eine Bewegung der Beineinheit 102b, insbesondere der unteren Beinstütze 126b, relativ zu der Fußeinheit 142b, insbesondere zu dem Gelenkgrundkörper 224b, zu ermöglichen.

Das Kopplungselement 222b ist als ein Poka-Yoke-Element ausgebildet. Das Kopplungselement 222b weist entlang einer Umfangsrichtung angeordnete Ausrichtungsfortsätze 236b, 238b auf. Eine ungerade Anzahl von Ausrichtungsfortsätzen 238b, insbesondere ein einzelner Ausrichtungsfortsatz 238b, ist verschieden von übrigen Ausrichtungsfortsätzen 236b ausgebildet. Im vorliegenden Ausführungsbeispiel weist das Kopplungselement 222b beispielhaft einen einzelnen Ausrichtungsfortsatz 238b auf, der verschieden von sieben übrigen Ausrichtungsfortsätzen 236b ausgebildet ist. Das Kopplungselement 222b weist, insbesondere entlang der Umfangsrichtung, eine blumenartige Formgebung auf, wobei insbesondere die Ausrichtungsfortsätze 236b, 238b gedachten Blütenblättern einer Blume entsprechen. Die, insbesondere blumenartige, Formgebung des Kopplungselements 222b, insbesondere die Ausrichtungsfortsätze 236b, 238b, bildet/bilden eine Verdrehsicherung des Kopplungselements 222b relativ zu der Gelenkeinheit 218b, insbesondere zu dem Gelenkgrundkörper 224b. Die Ausrichtungsfortsätze 236b, 238b weisen eine organische Form auf. Die Ausrichtungsfortsätze 236b, 238b sind frei von scharfen Kanten und/oder Ecken, insbesondere abgerundet, ausgebildet. Das Kopplungselement 222b, insbesondere die Ausrichtungsfortsätze 236b, 238b, ist/sind durch die Formgebung dazu vorgesehen, Verspannungen zwischen dem Kopplungselement 222b und der Gelenkeinheit 218b, insbesondere dem Gelenkgrundkörper 224b, gering zu halten.

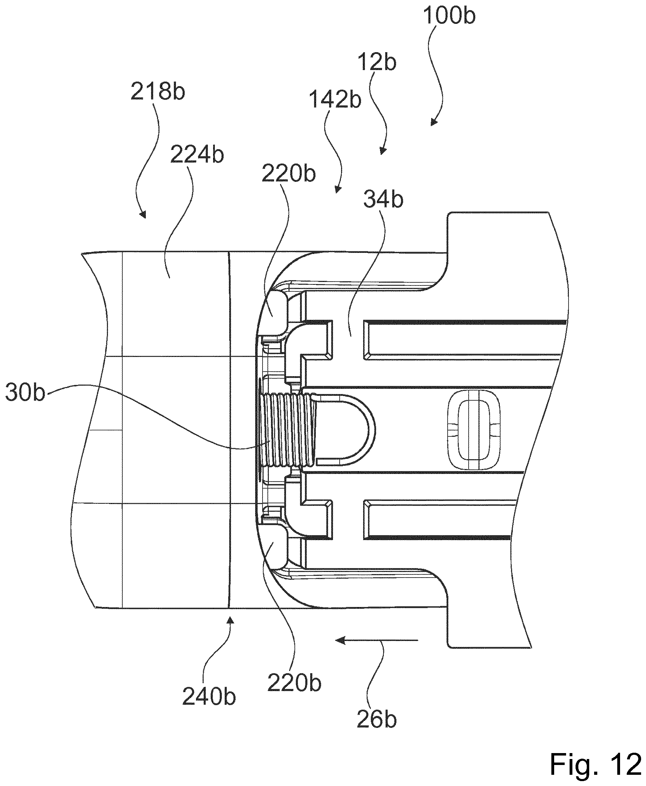

Figur 12 zeigt einen Teil der tragbaren Sitzhaltungshilfevorrichtung 100b aus Fig. 11 in einer weiteren schematischen Ansicht. Dargestellt ist ein Verbindungsbereich 240b zwischen der Gelenkeinheit 218b und eines Teils der Fußeinheit 142b und/oder der Lagereinheit 12b. Die Fußeinheit 142b weist zumindest ein Pufferelement 220b auf, das zu einer Aufpralldämpfung zumindest eines mit einer Rückstellkraft beaufschlagten, insbesondere kontrahierenden, Teils der Fußeinheit 142b und/oder der Lagereinheit 12b vorgesehen ist. Die Fußeinheit 142b weist eine Mehrzahl von Pufferelementen 220b auf. Die Fußeinheit 142b weist zumindest zwei Pufferelemente 220b auf, die insbesondere entlang einer zumindest im Wesentlichen senkrecht zu der Kontraktionsrichtung 26b verlaufenden Richtung beabstandet voneinander, insbesondere an dem Gelenkgrundkörper 224b, angeordnet sind. Im vorliegenden Ausführungsbeispiel weist die Fußeinheit 142b beispielhaft genau zwei Pufferelemente 220b auf. Die Pufferelemente 220b sind mit der Gelenkeinheit 218b verbunden, insbesondere einstückig mit dem Gelenkgrundkörper 224b ausgebildet. Die Pufferelemente 220b sind gummielastisch ausgebildet. Die Pufferelemente 220b sind aus demselben Material wie der Gelenkgrundkörper 224b, insbesondere aus einem Elastomer, ausgebildet. Alternativ ist vorstellbar, dass die Pufferelemente 220b aus einem von einem Material des Gelenkgrundkörpers 224b verschiedenen Material ausgebildet sind. Die Pufferelemente 220b sind in dem Verbindungsbereich 240b des Gelenkgrundkörpers 224b mit einem Teil der Fußeinheit 142b, insbesondere mit der Fußeinheitsstütze 148b, und/oder mit der Lagereinheit 12b angeordnet. Die Pufferelemente 220b dienen als Endanschlag eines weiteren Lagerelements 34b der Lagereinheit 12b in einer ersten Lagerstellung der Fußeinheit 142b. Die Pufferelemente 220b sind dazu vorgesehen, einen Aufprall des weiteren Lagerelements 34b, das von einem Rückstellelement 30b der Lagereinheit 12b mit einer Rückstellkraft beaufschlagt und insbesondere entlang der Kontraktionsrichtung 26b beschleunigt ist, auf den Gelenkgrundkörper 224b zu dämpfen. Die Pufferelemente 220b weisen stoßdämpfende Eigenschaften auf. Die Pufferelemente 220b sind durch eine infolge des Aufpralls des weiteren Lagerelements 34b auf die Pufferelemente 220b wirkende Kraft elastisch verformbar ausgebildet.

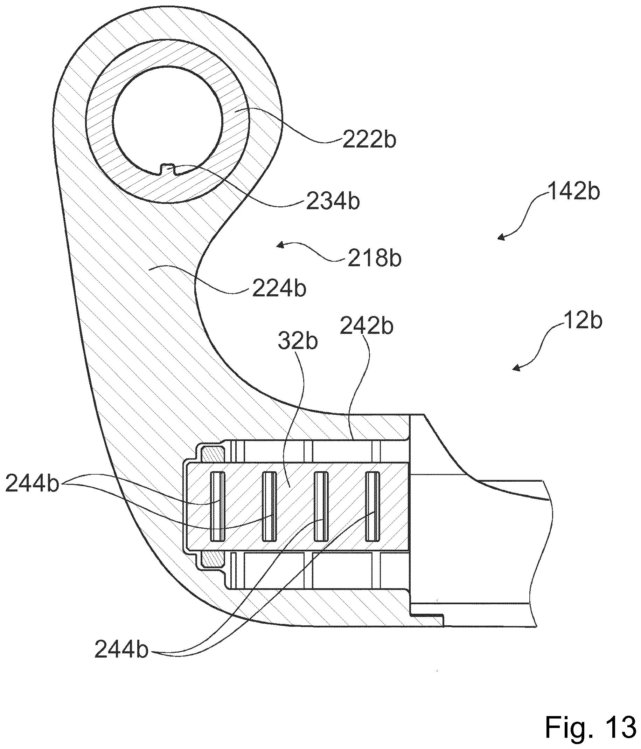

Figur 13 zeigt einen Teil der tragbaren Sitzhaltungshilfevorrichtung 100b aus Fig. 11 in einer schematischen Schnittansicht. Dargestellt sind die Gelenkeinheit 218b sowie ein Teil der Fußeinheit 142b und ein Teil der Lagereinheit 12b. Die Gelenkeinheit 218b ist spielfrei mit zumindest einem Teil der Fußeinheit 142b und/oder der Lagereinheit 12b verbunden, insbesondere verpresst. Die Gelenkeinheit 218b, insbesondere der Gelenkgrundkörper 224b, ist formschlüssig mit zumindest einem Teil der Fußeinheit 142b und/oder der Lagereinheit 12b verbunden. Die Gelenkeinheit 218b, insbesondere der Gelenkgrundkörper 224b, ist mit einem Lagerelement 32b und/oder mit einer Führungsschiene der Lagereinheit 12b spielfrei verbunden, insbesondere verpresst. Im vorliegenden Ausführungsbeispiel ist die Gelenkeinheit 218b, insbesondere der Gelenkgrundkörper 224b, beispielhaft mit dem Lagerelement 32b der Lagereinheit 12b spielfrei verbunden, insbesondere verpresst. Alternativ oder zusätzlich ist vorstellbar, dass die Gelenkeinheit 218b, insbesondere der Gelenkgrundkörper 224b, mit zumindest einem Teil der Fußeinheit 142b, insbesondere mit der Fußeinheitsstütze 148b, spielfrei verbunden, insbesondere verpresst, ist. Das Lagerelement 32b und/oder die Führungsschiene, im vorliegenden Ausführungsbeispiel insbesondere das Lagerelement 32b, sind/ist in den Gelenkgrundkörper 224b, insbesondere in eine von dem Gelenkgrundkörper 224b begrenzte Aufnahmeöffnung 242b, eingepresst. Das Lagerelement 32b und/oder die Führungsschiene, im vorliegenden Ausführungsbeispiel insbesondere das Lagerelement 32b, weisen/weist zumindest abschnittsweise, insbesondere zumindest in einem sich innerhalb des Gelenkgrundkörpers 224b erstreckenden Abschnitts, eine Tannenbaumgeometrie, insbesondere zu einer Realisierung einer Pressverbindung mit dem Gelenkgrundkörper 224b, auf. Das Lagerelement 32b und/oder die Führungsschiene, im vorliegenden Ausführungsbeispiel insbesondere das Lagerelement 32b, weisen/weist an dem sich innerhalb des Gelenkgrundkörpers 224b erstreckenden Abschnitts, insbesondere rippenartige, Fortsätze 244b zu einer Realisierung einer spielfreien, insbesondere verdrehsicheren, Verbindung mit dem Gelenkgrundkörper 224b auf. Die Fortsätze 244b bilden die Tannenbaumgeometrie des Lagerelements 32b und/oder der Führungsschiene, im vorliegenden Ausführungsbeispiel insbesondere des Lagerelements 32b, aus. Die Fortsätze 244b sind poka-yoke-artig, insbesondere zu einer Realisierung einer vorbestimmten Orientierung der Lagereinheit 12b relativ zu der Gelenkeinheit 218b, insbesondere zu dem Gelenkgrundkörper 224b, angeordnet.

## Patentansprüche

1. Tragbare Sitzhaltungshilfevorrichtung (100a; 100b) mit zumindest einer Beineinheit (102a; 102b, 104a), welche zumindest eine Beinlängsachse (110a, 128a; 128b) definiert, und mit zumindest einer Fußeinheit (142a; 142b), welche zur Anbindung eines Schuhs (214a) und/oder eines Fußes einer Person (200a) vorgesehen ist, wobei die Fußeinheit (142a; 142b) zumindest einen Schuhadapter (143a) und eine Schuhbindung (144a) umfasst, welche zumindest quer zu der zumindest einen Beinlängsachse (110a, 128a; 128b) miteinander koppelbar sind, **gekennzeichnet durch** eine Schnellkopplung (38a), welche dazu vorgesehen ist, den Schuhadapter (143a) und die Schuhbindung (144a) miteinander werkzeuglos zu koppeln, wobei die Schnellkopplung (38a) zumindest ein Rastelement (44a) umfasst, welches zumindest teilweise zur Ausbildung einer Kopplung des Schuhadapters (143a) mit der Schuhbindung (144a) vorgesehen ist.

2. Tragbare Sitzhaltungshilfevorrichtung (100a; 100b) nach Anspruch 1, **dadurch gekennzeichnet, dass** die Beineinheit (102a; 102b, 104a) zumindest eine Beinbeugungsebene (16a) definiert, relativ zu welcher der Schuhadapter (143a) und die Schuhbindung (144a) zumindest im Wesentlichen senkrecht miteinander koppelbar sind.

3. Tragbare Sitzhaltungshilfevorrichtung (100a; 100b) nach Anspruch 1 oder 2, **dadurch gekennzeichnet, dass** die Schnellkopplung (38a) zumindest zu einer kraft- und/oder formschlüssigen Kopplung von Schuhadapter (143a) und Schuhbindung (144a) vorgesehen ist.

4. Tragbare Sitzhaltungshilfevorrichtung (100a; 100b) nach einem der vorhergehenden Ansprüche, **dadurch gekennzeichnet, dass** die Schnellkopplung (38a) zumindest ein Magnetelement (48a) umfasst, welches zumindest teilweise wenigstens zur Einleitung einer Kopplung des Schuhadapters (143a) mit der Schuhbindung (144a) vorgesehen ist.

5. Tragbare Sitzhaltungshilfevorrichtung (100a; 100b) nach Anspruch 4, **dadurch gekennzeichnet, dass** das Magnetelement (48a) dazu vorgesehen ist, eine Kopplung des Schuhadapters (143a) mit der Schuhbindung (144a) mittels des Rastelements (44a) auszulösen.

6. Tragbare Sitzhaltungshilfevorrichtung (100a; 100b) nach einem der vorhergehenden Ansprüche, **dadurch gekennzeichnet, dass** die Schnellkopplung (38a) den Schuhadapter (143a) und die Schuhbindung (144a) um eine Drehachse (52a) drehbar quer zu der zumindest einen Beinlängsachse (110a, 128a; 128b) lagert.

7. Tragbare Sitzhaltungshilfevorrichtung (100a; 100b) nach einem der vorhergehenden Ansprüche, **gekennzeichnet durch** zumindest eine Schnelllöseeinheit (54a), welche dazu vorgesehen ist, den Schuhadapter (143a) und die Schuhbindung (144a) voneinander werkzeuglos zu lösen.

8. Tragbare Sitzhaltungshilfevorrichtung (100a; 100b) nach Anspruch 7, **dadurch gekennzeichnet, dass** die Schnelllöseeinheit (54a) zumindest ein Bedienelement (56a) zur Entkopplung des Schuhadapter (143a) und der Schuhbindung (144a) voneinander aufweist.

9. Tragbare Sitzhaltungshilfevorrichtung (100a; 100b) nach Anspruch 8, **dadurch gekennzeichnet, dass** das Bedienelement (56a) bei einer Betätigung zumindest mittelbar das Rastelement (44a) relativ zu dem Magnetelement (48a) verschiebt.

10. Tragbare Sitzhaltungshilfevorrichtung (100a; 100b) nach Anspruch 8 oder 9, **dadurch gekennzeichnet, dass** das Bedienelement (56a) zumindest im Wesentlichen parallel zu der zumindest einen Beinlängsachse (110a, 128a; 128b) betätigbar ist.

11. Verfahren zum Betrieb einer tragbaren Sitzhaltungshilfevorrichtung (100a; 100b) nach einem der Ansprüche 1 bis 10, **dadurch gekennzeichnet, dass** in zumindest einem Verfahrensschritt (300a, 302a, 304a, 306a, 308a) der Schuhadapter (143a) und die Schuhbindung (144a) zumindest quer zu der zumindest einen Beinlängsachse (110a, 128a; 128b) der Beineinheit (102a; 102b, 104a) miteinander gekoppelt werden, wobei die Schnellkopplung (38a) den Schuhadapter (143a) und die Schuhbindung (144a) miteinander werkzeuglos koppelt, wobei das zumindest eine Rastelement (44a) der Schnellkopplung (38a) zumindest teilweise die Kopplung des Schuhadapters (143a) mit der Schuhbindung (144a) ausbildet.

## Claims

1. Wearable sitting-posture assisting device (100a; 100b)
with at least one leg unit (102a; 102b; 104a) that defines at least one longitudinal leg axis (110a, 128a; 128b)
and with at least one foot unit (142a; 142b) that is configured for a connection of a shoe (214a) and/or a foot of a person (200a), wherein the foot unit (142a; 142b) comprises at least one shoe adapter (143a) and at least one shoe connector (144a) which are couplable with one another at least transversely to the at least one at least one longitudinal leg axis (110a, 128a; 128b),
**characterized by** a quick coupling (38a) which is configured for a tool-less coupling of the shoe adapter (143a) and the shoe connector (144a) with each other,
wherein the quick coupling (38a) comprises at least one latch element (44a) that is configured at least partially to create a coupling of the shoe adapter (143a) with the shoe connector (144a).

2. Wearable sitting-posture assisting device (100a; 100b) according to claim 1, **characterized in that** the leg unit (102a; 102b; 104a) defines at least one leg bending plane (16a), relative to which the shoe adapter (143a) and the shoe connector (144a) are couplable at least substantially orthogonally.

3. Wearable sitting-posture assisting device (100a; 100b) according to claim 1 or 2, **characterized in that** the quick coupling (38a) is configured at least for a force-fit and/or form-fit coupling of the shoe adapter (143a) and the shoe connector (144a).

4. Wearable sitting-posture assisting device (100a; 100b) according to one of the preceding claims,
**characterized in that** the quick coupling (38a) comprises at least one magnet element (48a), which is configured at least partially at least for an initiation of a coupling of the shoe adapter (143a) with the shoe connector (144a).

5. Wearable sitting-posture assisting device (100a; 100b) according to claim 4, **characterized in that** the magnet element (48a) is configured for activating a coupling of the shoe adapter (143a) with the shoe connector (144a) via the latch element (44a).

6. Wearable sitting-posture assisting device (100a; 100b) according to one of the preceding claims,
**characterized in that** the quick coupling (38a) supports the shoe adapter (143a) and the shoe connector (144a) rotatably around a rotation axis (52a) transversely to the at least one longitudinal leg axis (110a, 128a; 128b).

7. Wearable sitting-posture assisting device (100a; 100b) according to one of the preceding claims,
**characterized by** at least one quick release unit (54a), which is configured to release the shoe adapter (143a) and the shoe connector (144a) from each other without a tool.

8. Wearable sitting-posture assisting device (100a; 100b) according to claim 7, **characterized in that** the quick release unit (54a) comprises at least one actuation element (56a) for a decoupling of the shoe adapter (143a) and the shoe connector (144a) from each other.

9. Wearable sitting-posture assisting device (100a; 100b) according to claim 8, **characterized in that** in an actuation the actuation element (56a) displaces the latch element (44a) relative to the magnet element (48a) at least indirectly.

10. The wearable sitting-posture assisting device (100a; 100b) according to claim 8 or 9,
**characterized in that** the actuation element (56a) can be actuated at least substantially parallel to the at least one longitudinal leg axis (110a, 128a; 128b).

11. Method for an operation of a wearable sitting-posture assisting device (100a; 100b) according to one of claims 1 to 10,
**characterized in that** in at least one method step (300a, 302a, 304a, 306a, 308a) the shoe adapter (143a) and the shoe connector (144a) are coupled with each other at least transversely to the at least one longitudinal leg axis (110a, 128a; 128b) of the leg unit (102a; 102b; 104a),
wherein the quick coupling (38a) couples the shoe adapter (143a) and the shoe connector (144a) with each other without a tool,
wherein the at least one latch element (44a) of the quick coupling (38a) at least partially realizes the coupling o2f the shoe adapter (143a) with the shoe connector (144a).

## Revendications

1. Dispositif portable d'aide à la position assise (100a ; 100b)
avec au moins une unité de jambe (102a ; 102b, 104a) qui définit au moins un axe longitudinal de jambe (110a, 128a ; 128b),
et avec au moins une unité de pied (142a ; 142b) prévue pour relier une chaussure (214a) et/ou un pied d'une personne (200a),
l'unité de pied (142a ; 142b) comportant au moins un adaptateur-chaussure (143a) et une fixation de chaussure (144a) qui peuvent être couplés l'un à l'autre au moins transversalement à l'au moins un axe longitudinal de jambe (110a, 128a ; 128b),
**caractérisé par** un raccordement rapide (38a) prévu pour coupler l'adaptateur-chaussure (143a) et la fixation de chaussure (144a) l'un à l'autre sans outil,
le raccordement rapide (38a) comprenant au moins un élément d'encliquetage (44a) prévu au moins en partie pour réaliser un accouplement de l'adaptateur-chaussure (143a) avec la fixation de chaussure (144a).

2. Dispositif portable d'aide à la position assise (100a ; 100b) selon la
revendication 1,
**caractérisé en ce que** l'unité de jambe (102a ; 102b, 104a) définit au moins un plan de flexion de jambe (16a) par rapport auquel l'adaptateur-chaussure (143a) et la fixation de chaussure (144a) peuvent être couplés l'un à l'autre au moins sensiblement perpendiculairement.

3. Dispositif portable d'aide à la position assise (100a ; 100b) selon la
revendication 1 ou 2,
**caractérisé en ce que** le raccordement rapide (38a) est prévu au moins pour un accouplement par liaison de force et/ou de forme de l'adaptateur-chaussure (143a) et de la fixation de chaussure (144a).

4. Dispositif portable d'aide à la position assise (100a ; 100b) selon l'une des revendications précédentes,
**caractérisé en ce que** le raccordement rapide (38a) comprend au moins un élément magnétique (48a) prévu au moins en partie au moins pour démarrer un accouplement de l'adaptateur-chaussure (143a) avec la fixation de chaussure (144a).

5. Dispositif portable d'aide à la position assise (100a ; 100b) selon la
revendication 4,
**caractérisé en ce que** l'élément magnétique (48a) est prévu pour déclencher un accouplement de l'adaptateur-chaussure (143a) avec la fixation de chaussure (144a) par l'élément d'encliquetage (44a).

6. Dispositif portable d'aide à la position assise (100a ; 100b) selon l'une des revendications précédentes,
**caractérisé en ce que** le raccordement rapide (38a) supporte l'adaptateur-chaussure (143a) et la fixation de chaussure (144a) de manière rotative autour d'un axe de rotation (52a) transversalement à l'au moins un axe longitudinal de jambe (110a, 128a ; 128b).

7. Dispositif portable d'aide à la position assise (100a ; 100b) selon l'une des revendications précédentes,
**caractérisé par** au moins une unité de détachement rapide (54a) prévue pour détacher l'adaptateur-chaussure (143a) et la fixation de chaussure (144a) l'un de l'autre sans outil.

8. Dispositif portable d'aide à la position assise (100a ; 100b) selon la
revendication 7,
**caractérisé en ce que** l'unité de détachement rapide (54a) comprend au moins un élément de commande (56a) pour découpler l'adaptateur-chaussure (143a) et la fixation de chaussure (144a) l'un de l'autre.

9. Dispositif portable d'aide à la position assise (100a ; 100b) selon la revendication 8,
**caractérisé en ce que** l'élément de commande (56a) lors d'un actionnement déplace au moins indirectement l'élément d'encliquetage (44a) par rapport à l'élément magnétique (48a).

10. Dispositif portable d'aide à la position assise (100a ; 100b) selon la
revendication 8 ou 9,
**caractérisé en ce que** l'élément de commande (56a) peut être actionné au moins sensiblement en parallèle à l'au moins un axe longitudinal de jambe (110a, 128a ; 128b).

11. Procédé de fonctionnement d'un dispositif portable d'aide à la position assise (100a ; 100b) selon l'une des revendications 1 à 10,
**caractérisé en ce que** dans au moins une étape de procédé (300a, 302a, 304a, 306a, 308a), l'adaptateur-chaussure (143a) et la fixation de chaussure (144a) sont couplés l'un à l'autre au moins transversalement à l'au moins un axe longitudinal de jambe (110a, 128a ; 128b) de l'unité de jambe (102a ; 102b, 104a), le raccordement rapide (38a) couplant l'adaptateur-chaussure (143a) et la fixation de chaussure (144a) l'un à l'autre sans outil,
où l'au moins un élément d'encliquetage (44a) du raccordement rapide (38a) réalise au moins en partie l'accouplement de l'adaptateur-chaussure (143a) avec la fixation de chaussure (144a).
